# EUROPEAN PATENT SPECIFICATION

(11) **EP 4 181 810 B1**
(45) Date of publication and mention of the grant of the patent: **26.08.2026**
(21) Application number: 21845872.7
(22) Date of filing: 19.07.2021
(51) Int. Cl.: A61M 25/01, A61B 18/14, A61B 90/00, A61B 17/00, A61B 18/00, A61M 25/09, A61B 17/34, A61M 29/00

(54) **HYBRID TRANSSEPTAL DILATOR**
HYBRIDER TRANSSEPTALER DILATATOR
DILATATEUR TRANSSEPTAL HYBRIDE

(30) Priority: 20.07.2020 US 202063053930 P; 30.09.2020 US 202063085517 P
(43) Date of publication of application: 24.05.2023
(73) Proprietor: Boston Scientific Medical Device Limited, Galway H91 Y868 (IE)
(72) Inventor: LEUNG, Charlene, Mississauga, Ontario L4W 5P6 (CA); MOK, Daniel Wing Fai, Mississauga, Ontario L4W 5P6 (CA); MORIYAMA, Eduardo, Mississauga, Ontario L4W 5P6 (CA); DAVIES, Gareth, Mississauga, Ontario L4W 5P6 (CA); LOUGHEED, Olivia, Mississauga, Ontario L4W 5P6 (CA); SULLIVAN, Dermot, Mississauga, Ontario L4W 5P6 (CA); LEUNG, Linus, Mississauga, Ontario L4W 5P6 (CA); BUDD, Ryan, Mississauga, Ontario L4W 5P6 (CA)
(74) Representative: Pfenning, Meinig & Partner mbB
(86) International application number: PCT/IB2021/056514
(87) International publication number: WO 2022/018617

(56) References cited:
- US-A1- 2010 022 948
- US-A1- 2010 022 948
- US-A1- 2012 123 327
- US-A1- 2015 045 696
- US-A1- 2018 093 068
- US-A1- 2019 015 644
- US-A1- 2019 015 644
- US-A1- 2019 029 722
- US-A1- 2019 160 266
- US-A1- 2020 060 710

## Description

### TECHNICAL FIELD

The present disclosure relates to a medical device for use in accessing the cardiovascular system. More particularly the present disclosure relates to a hybrid transseptal dilator for facilitating a transseptal procedure for providing left heart access.

### BACKGROUND

When performing a transseptal procedure to gain access to the left atrium of a heart, a physician typically uses a sheath and dilator to support a crossing or puncturing device. In some cases, a physician may not be able to cross through to the left atrium as the transition between sheath and dilator may get stuck or snag at the tissue boundary, and as a result the sheath may not be able to cross through the perforation (or it crosses with difficulty). In other words, the tissue may get hung up at the sheath/dilator interface. Thus, the use of multiple devices in a transseptal procedure may make it difficult for the operator to complete the procedure due to the material transitions between various devices which may get caught at the septal tissue interface.

Some conventional transseptal procedures, for example some that use the inferior approach to gain access to the heart, use a needle in order to carry out a transseptal puncture. Certain limitations may be associated with the use of needles or other rigid devices for carrying out a transseptal puncture procedure.

These limitations may include one or more of: (1) need for a separate exchange wire to gain access to the SVC resulting in multiple device exchanges on the right side; (2) the use of a needle may require multiple device exchanges in order to complete the procedure; (3) difficulty in correcting placement of the puncture device after insertion within the right atrium if the target site on the septum is missed; (4) there may be a lack of repeatability for certain aspects of the procedure for completing the puncture in an effective and timely manner; (5) the puncture device may not provide sufficient atraumacity and may result in excessive force being applied to puncture tissue resulting in damage to tissue; (6) possible risk of trauma to the structures within the left atrium following puncture due to the force of advancement; (7) there may be a lack of adequate anchoring after puncture to maintain access; (8) need for an additional exchange on the left side requiring removal of the puncture device and advancement of another wire (such as a pigtail wire) to facilitate anchoring; and/or (9) trackability to allow additional devices to be tracked over the wire once in the left side.

Document US 2012/123327 A1 describes a steerable endoluminal device adapted for delivery into a patient's vasculature. The device includes a tubular member having a distal deflection portion that extends to the distal end and a main body portion that extends from the deflectable portion to the proximal end. The tubular member further includes a stiff portion extending along the distal deflection portion, and being formed of polymeric material, which is disposed circumferentially adjacent to the stiff portion. The stiff portion is made of a material that has an elastic modulus greater than the elastic modulus of the polymeric material. A pull wire extends between the proximal end and the distal end of the tubular member and is attached to the distal deflection portion to control deflection of the distal deflection portion of the tubular member.

Document US 2010/022948 A1 describes a catheter that is configured to extend through a transseptal puncture site, a left atrium, and into a left ventricle. The catheter may comprise a shaft defining at least one lumen configured to receive at least one of an implant and/or a dilator, a first steerable actuator coupled to the shaft at a position on the shaft substantially corresponding to the annulus of the mitral valve when the distal end of the shaft is disposed within the left ventricle, and a handle assembly coupled to a proximal end of the shaft. The handle assembly may comprise a first actuator coupled to the first steerable actuator. The first actuator may be configured to apply a force to the first steerable actuator to deflect the shaft about a region proximate to the first steerable actuator.

Document US 2019/015644 A1 describes a method and apparatus for an optimized transseptal procedure for providing left heart access, that reduces the number of devices that are used in order to minimize procedural time, complexity and cost. The apparatus comprises a hybrid dilator that comprises the combined functionality of a transseptal sheath and dilator assembly. The hybrid dilator comprises: a dilator shaft defining a lumen for receiving a crossing device therethrough, the dilator shaft being structured to provide support for the crossing device when the crossing device is used to create a puncture in a tissue; and a distal tip having an outer diameter which tapers down to an outer diameter of the crossing device for providing a smooth transition between the crossing device and the distal tip when the crossing device is inserted through the lumen and protrudes beyond the distal tip.

Document US 2015/045696 A1 describes a steerable dilator includes a dilator body for holding and guiding a sheath, the dilator body having a deflectable distal end portion, and at least one lateral passage configured to accommodate at least one steering cable and a steering handle operatively associated with a proximal end portion of the dilator and having an actuation mechanism operatively connected to the at least one steering cable accommodated within the at least one lateral passages of the dilator for steering the deflectable distal end portion of the dilator in at least one direction.

Document US 2019/029722 A1 describes a transseptal insertion device for transseptal puncture of a cardiac interatrial septum. The device includes a sheath that defines a lumen and has a distal end that is closest to the cardiac interatrial septum of a patient and a proximal end that is external to the patent, a balloon that is connected to the distal end of the sheath, in which the balloon, when inflated, overhangs and extends past the distal end of the sheath, preventing accidental puncturing of the cardiac interatrial septum and stabilizing the transseptal insertion device against fossa ovalis of the cardiac interatrial septum, and a dilator that is positioned within the at least one lumen. The dilator has a distal end and is designed to and is capable of puncturing the cardiac interatrial septum without the use of a needle or other sharp instrument.

### BRIEF DESCRIPTION OF THE DRAWINGS

In order that the invention may be readily understood, embodiments of the invention are illustrated by way of examples in the accompanying drawings 14A , 14B, 15A, and 15B with the rest of the drawings being useful for the understanding of the invention, in which:
Fig. 1A is an illustration of a hybrid dilator, in accordance with an embodiment of the present disclosure;
Fig. 1B is an illustration of a proximal portion of the hybrid dilator of Fig. 1A;
Fig. 1C is a front end view of a distal tip of the hybrid dilator of Fig. 1A;
Fig. 1D is an illustration of a proximal portion of the hybrid dilator of Fig. 1A
Fig. 2A. is a cross-sectional view of the distal tip of a hybrid dilator taken along the lines 2A-2A of Fig. 1C;
Fig. 2B. is a cross-sectional view of the distal most end of a hybrid dilator taken along the lines 2B-2B of Fig. 1C;
Fig. 2C is an illustration of a distal tip, in accordance with an alternative embodiment of the present disclosure;
Figs. 3A-3D illustrate alternate embodiments of a distal tip, in accordance with alternate embodiments of the present disclosure;
Fig. 4A illustrates a hybrid dilator in accordance with an embodiment of the present disclosure, and a standard sheath/dilator assembly usable in a standard transseptal procedure;
Figs. 4B-4G illustrate a proximal portion of the hybrid dilator in accordance with an embodiment of the present disclosure;
Figs. 5A-5C illustrates a proximal portion of a hybrid dilator, in accordance with an alternate embodiment of the present disclosure;
Fig. 5D illustrates a hybrid dilator, in accordance with an alternate embodiment of the present disclosure;
Fig. 5E illustrates an alternative embodiment of a proximal hub, in accordance with an embodiment of the present disclosure;
Fig. 6A is an illustration of a method of using a sheath and dilator, in accordance with a standard transseptal procedure;
Fig. 6B is a flowchart illustrating steps in a standard transseptal procedure;
Fig. 7A is an illustration of a method for performing a transseptal puncture procedure using a hybrid dilator, in accordance with an embodiment of the present disclosure;
Fig. 7B is a flowchart illustrating steps of a method for performing a transseptal puncture procedure using a hybrid dilator, in accordance with an embodiment of the present disclosure;
Fig. 8 is a cross sectional view of the shaft and distal tip of a hybrid dilator of an alternative embodiment of the present disclosure;
Fig. 9 is an enlarged view of the distal tip of Fig. 8;
Fig. 10A-10D illustrates a cross sectional view of the shaft of a hybrid dilator in accordance with an embodiment of the present disclosure;
Fig. 11A-11D illustrates a cross sectional view of the shaft of a hybrid dilator in accordance with an alternate embodiment of the present disclosure;
Fig. 12 is a flowchart illustrating steps of a method for performing a transseptal puncture procedure using a hybrid dilator in accordance with an embodiment of the present disclosure;
Fig. 13A is an illustration of a steerable hybrid dilator, in accordance with an embodiment of the present disclosure;
Fig. 13B is an illustration of the handle of the embodiment of Fig. 13A;
Fig. 14 A illustrates a hybrid dilator in accordance with an embodiment of the present invention;
Fig. 14B illustrates the hybrid dilator of 14A in use;
Fig. 15A illustrates a hybrid dilator in accordance with an alternate embodiment of the present invention;
Fig. 15B illustrates the hybrid dilator of 15A in use;
Fig. 16A illustrates a hybrid dilator in accordance with an alternate embodiment of the present disclosure;
Fig. 16B illustrates the hybrid dilator of 16B in use;
Fig. 17 is a flowchart illustrating steps of a method for performing a transseptal puncture procedure using a hybrid dilator in accordance with an alternate embodiment of the present disclosure;
Fig. 18 illustrates a flexible puncture device in accordance with an embodiment of the present disclosure;
Fig. 19 illustrates the distal tip of a hybrid dilator with a radiopaque marker in accordance with an alternate embodiment of the present disclosure;
Fig. 20 illustrates the distal tip of a hybrid dilator with a radiopaque marker under fluoroscopy; and
Fig. 21 illustrates the mechanical properties of Nitinol compared to steel;
Fig. 22A- 22B illustrate a hybrid dilator in accordance with an alternate embodiment of the present disclosure;
Fig 22C- 22D illustrate a cross sectional view of the hybrid dilator of Fig. 22A;
Fig. 23A - 23C illustrate the distal portion of a hybrid dilator in accordance with an embodiment of the present disclosure;
Fig. 24 A- 24C illustrate the distal portion of a hybrid dilator with a radiopaque marker in accordance with an alternate embodiment of the present disclosure; and
Fig. 25 illustrates the hub of a hybrid dilator in accordance with an embodiment of the present disclosure;
Fig. 26 is an illustration of a method of using a sheath and dilator, in accordance with a standard transseptal procedure;
Figs. 27A-29B are illustrations of a method of using a hybrid dilator in accordance with an embodiment of the present disclosure;
Fig. 30 is a flowchart illustrating steps of a method for performing a transseptal puncture procedure using a hybrid dilator in accordance with an embodiment of the present disclosure; Fig. 31 is a flowchart illustrating steps of a method for performing a transseptal puncture procedure using a hybrid dilator in accordance with an alternate embodiment of the present disclosure;

### DETAILED DESCRIPTION

The problem of a transseptal puncture being performed using a crossing device which is supported by a sheath and dilator set having a transition which may snag on tissue when crossing the septum, can be addressed by using a hybrid dilator (described herein) instead of the sheath and dilator set to thereby eliminate the transition, wherein the hybrid dilator has the appropriate functionality (flexibility, pushability, torqueability, distal taper, steerability, etc.) to facilitate a smooth crossing.

The inventors of the present invention have discovered systems and methods that attempt to overcome the limitations associated with prior art systems. The invention is defined in claim 1. Further aspects and preferred embodiments are defined in the dependent claims. Aspects, embodiments and examples of the present disclosure which do not fall under the scope of the appended claims do not form part of the claimed invention and are merely provided for illustrative purposes. In particular, surgical and therapeutical methods are not claimed.

In one broad aspect, embodiments include a hybrid dilator for use with a crossing device in tissue puncturing procedures, the hybrid dilator comprising: a dilator shaft defining a lumen for receiving a crossing device therethrough, the dilator shaft being structured to allow navigation to the target site and provide support for the crossing device when the crossing device is used to create a puncture in a tissue; and a distal tip having an outer diameter which tapers down to an outer diameter of the crossing device for providing a smooth transition between the crossing device and the distal tip when the crossing device is inserted through the lumen and protrudes beyond the distal tip. In some such embodiments, the dilator shaft comprises an inner layer, an outer layer, and a torque layer therebetween.

In the embodiments of the present invention, the hybrid dilator comprises a stiffening member that is reshapeable.

In the embodiments of the present invention, the hybrid dilator comprises a deflectable distal end.

In the embodiments of the present invention, the hybrid dilator is steerable.

Additionally, the present inventors have discovered a method to perform a transseptal medical procedure that streamlines the procedural workflow by providing a hybrid dilator that replaces a conventional transseptal sheath and dilator assembly. With the hybrid dilator of the present invention a reduced number of devices may be required in order to complete a transseptal procedure. This reduces the number of parts that a physician is required to prepare and assemble for the transseptal procedure and introduce into the patient. The present method provides a dilator that is usable with a guidewire for access that replaces a sheath, dilator, and guidewire assembly.

With specific reference now to the drawings in detail, it is stressed that the particulars shown are by way of example and for purposes of illustrative discussion of certain embodiments of the present invention only. Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

In some embodiments, a single piece/unitary device in the form of a hybrid dilator is provided that provides smooth tapers functions to facilitate both the crossing and the exchange of devices in a trans-septal procedure while still providing the physician with tactile feedback and distal curve indication that are substantially equivalent to those provided by a sheath/dilator assembly.

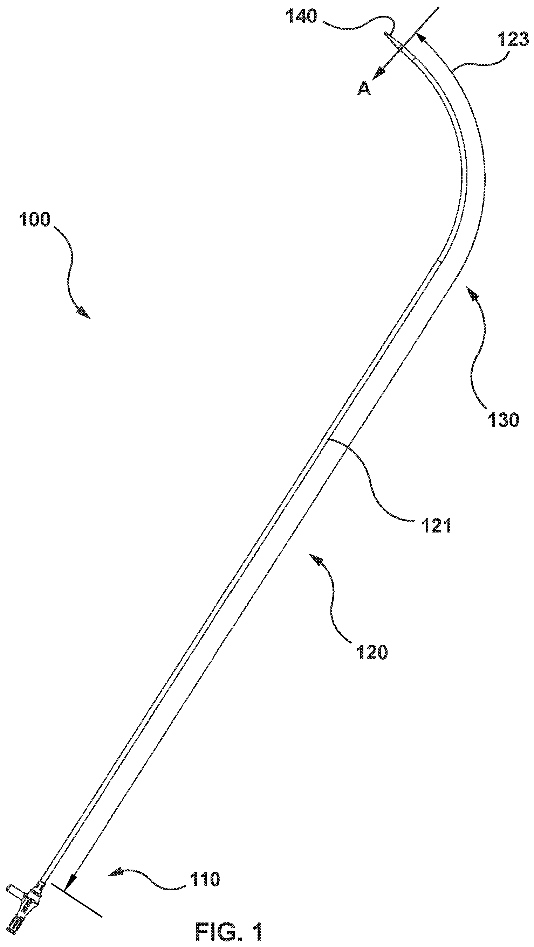

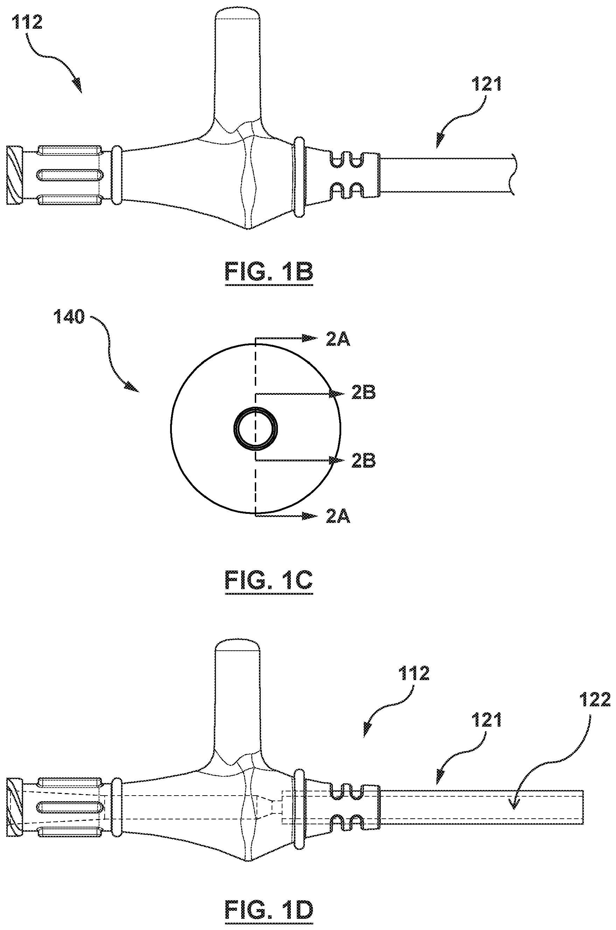

In accordance with an embodiment of the present disclosure, a hybrid dilator 100 is provided, as shown in Fig. 1A. The hybrid dilator 100 comprises a combination of features that provide a dual functionality of a sheath and a dilator for facilitating a transseptal puncture procedure while avoiding disadvantages of conventional sheath and dilator assemblies. The hybrid dilator 100 provides the smoothness of a standard transseptal dilator with the control of a standard transseptal sheath. More specifically, the hybrid dilator 100 functions as a single device that removes the need for using a conventional sheath/dilator assembly and eliminates the need for assembly, resulting in less waste, fewer exchanges, and reduced procedure time. The hybrid dilator 100 comprises a sheath-like handle with familiar torque and tactile control. In the specific example shown, the hybrid dilator 100 defines a proximal portion 110 comprising a molded combination proximal hub 112, as shown in Figs. 1B and 1D. A distal portion 120 is coupled to the proximal portion 110 comprising a dilator shaft. The dilator shaft extends from the proximal end and defines a curved distal end 130 that terminates in a distal tip 140, as additionally shown in Fig. 1C.

### Dilator shaft / Support and columnar strength/positioning

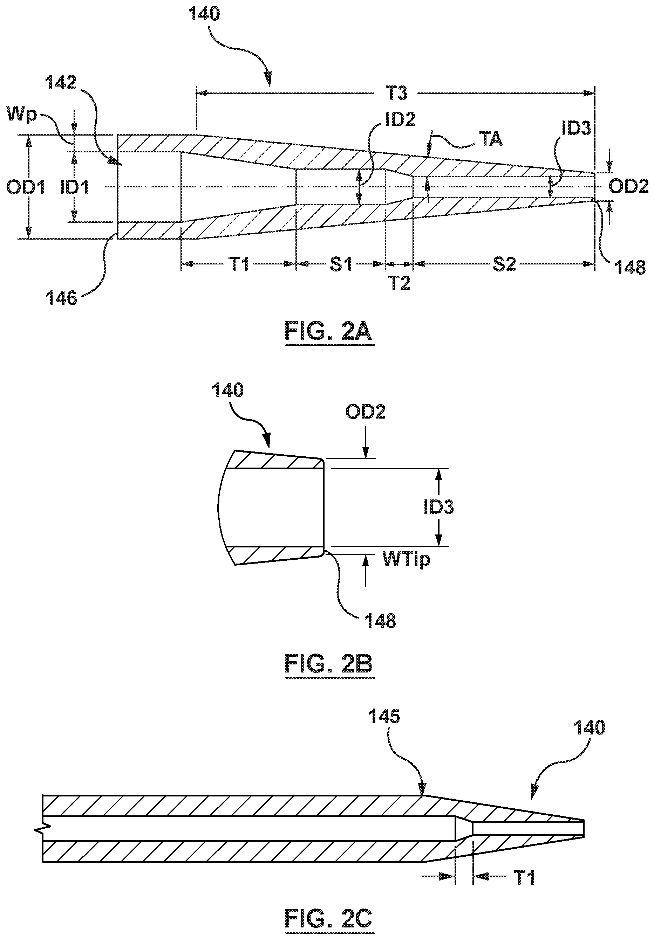

The dilator shaft is formed from a smooth distal tubing 121 that is coupled to the molded proximal hub 112. The distal tubing 121 defines a lumen 122 there-through that narrows at the distal tip 140 and which may be used to flush the device prior to use. In some embodiments, since the hybrid dilator 100 provided a single unitary device, this means that one product is to be flushed unlike the prior art sheath/dilator assembly where each product requires flushing. The dilator shaft provides mechanical properties to best facilitate procedural activities. At the distal tip 140, as illustrated further in Fig. 2A, the distal tubing 121 transitions through a smooth external taper T3 that widens in the proximal direction to a greater outer diameter (OD) than a conventional transseptal kit dilator so as to dilate the septum to an appropriate size for the subsequent delivery device or equipment that may be used. The OD of the distal tubing 121 is substantially constant from the proximal edge of distal tip 140 till the proximal hub 112 where the distal tubing is coupled or attached thereto. In some such embodiments, the OD of the hybrid dilator 100 may vary based on the application and clinical use. In some embodiments, the size of hybrid dilator 100 is from about 12 French to about 20 French. In a specific example, the hybrid dilator has a size of about 12.5 French (outer diameter of about 0.163 inches (0.414 cm) to about 0.166 inches (0.421 cm)). In another example, the hybrid dilator has a size of about 15 French (outer diameter of about 0.193 inches (0.490 cm) to about 0.205 inches (0.521 cm)).

### Distal end curvature

In some embodiments of the present disclosure, the distal end 130 of the hybrid dilator 100 may be curved as shown in Fig. 1A. Alternatively, the distal end 130 of the hybrid dilator may be straight. In some embodiments where the distal end 130 of the hybrid dilator 100 is curved, the hybrid dilator 100, in combination with a puncturing device such as a needle, forms a trajectory that is substantially equivalent to the trajectory achieved by the combination of a sheath/dilator/needle assembly of a conventional transseptal kit to provide physicians with a predictable and repeatable path for completing a transseptal puncture. The curved distal end 130 facilitates advancement of the hybrid dilator 100 in conjunction with the puncturing device to initiate a transseptal puncture.

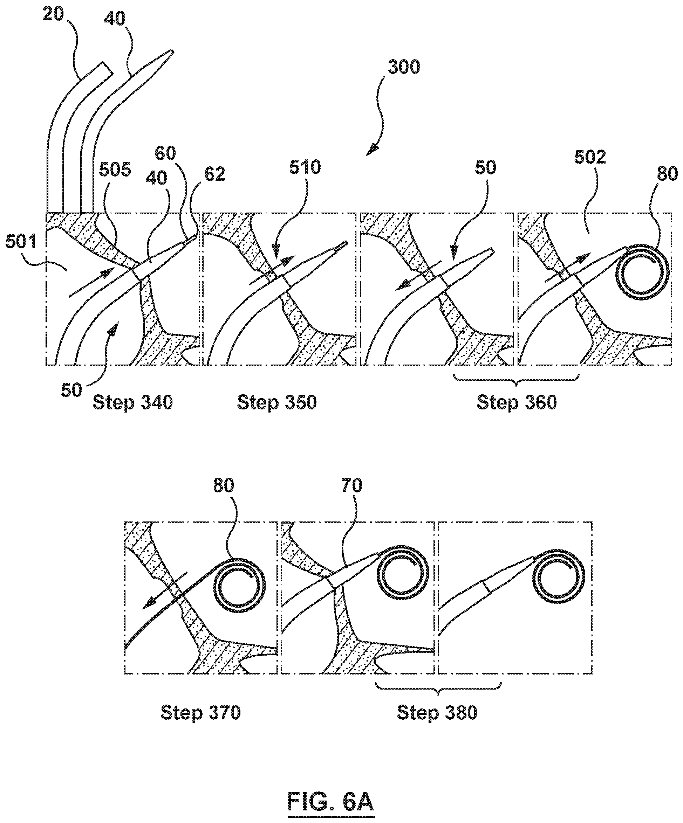

In some such embodiments, the hybrid dilator 100 comprises a shaft formed from distal tubing 121 that is sufficiently rigid to enable positioning of a crossing device such as a puncturing needle or a guidewire to be advanced through it while maintaining the position of the assembly at a desired site, such as a fossa of a septum. As such, the hybrid dilator 100 functions to provide support and columnar strength to facilitate placement of the crossing device at the desired location. As disclosed above and as shown in Figs. 1A, distal tubing 121 tapers proximally from the distal tip 140 to a greater OD defining a dilating interface to allow dilation of the puncture site 510 (Fig. 6A) to facilitate additional devices to be advanced there-through.

### Distal Tip

More specifically, in some embodiments as shown in Fig. 2A, the distal tip 140 provides a lumen 142 that is appropriate for a crossing device such as a puncturing device to be inserted there-through and defines a relatively thin wall to facilitate controlled puncture. In some such examples, the puncturing device is a mechanical needle or an RF puncturing device that is usable with the hybrid dilator 100. The hybrid dilator 100 provides a restricted distal internal diameter (as shown by ID2 and ID3) at the distal tip 140 to control the distance by which the puncture device such a transseptal needle (with a narrow distal portion) protrudes from the hybrid dilator 100. The narrowest distal portion of a compatible puncturing device has an outer diameter less than ID3 whereby it extends into and through length S2 of lumen 142, and beyond distal edge 148, while, typically, a part of the puncturing device having an outer diameter greater than ID3 and less than ID2 will be seated in internal taper T2. Consequently, the dimension of length S2 is significant in determining the distance the puncturing device protrudes from the hybrid dilator 1. In some such embodiments, this allows the hybrid dilator 100 to meet the same standard as existing transseptal dilators in that it controls the distance by which a transseptal needle can protrude when fully inserted therein. Additionally, as described previously, the distal tip 140 provides an external taper T3 that allows the dilator OD to transition from a narrow OD2 at a distal most end or distal edge 148 of the distal tip 140, to a wider OD1 at its proximal edge 146. In some such examples, the hybrid dilator 100 has smooth lines and a smooth external taper T3 to facilitate a seamless transition across tissue. In some such examples, the hybrid dilator 100 functions to reduce the number of physical or geometric transitions or material transitions which can cause difficulties and/or create tactile obstructions hindering a physician's ability to complete a transseptal or other tissue crossing.

In typical examples, as shown in Figs. 1A and 2A, the dilator shaft includes a distal tubing 121 which, in some examples, comprises a high density polyethylene (HDPE) tubing. In some such embodiments, the HDPE has a hardness from about 55 shore D to about 70 shore D, and in a specific example, the HDPE hardness is about 67 shore D. In typical embodiments, the distal tubing 121 comprises material that meets the functional requirements of a transseptal sheath/dilator kit. In some such examples, the distal tubing 121 comprises a straight shaft that transitions into curved distal end 130. The distal tip 140 comprises a tapered tip with a smooth external taper T3, having a taper angle TA of about 5.5° +/- 1° degrees, and internal geometry which provides a controlled internal diameter (ID) to provide a predicable needle extension length. In some embodiments, the length of the external taper T3 ranges from about 0.4 inches (1 cm) to about 1 inch (2.5 cm). In some such examples, the taper length for external taper T3 is equal to about 0.646" or about 1.6cm. The distal tubing 121 has an inner diameter ID1 that is equal to about 0.109" (0.277 cm) and an outer diameter OD1 that is equal to about 0.166" (0.422 cm) along its proximal portion (or proximal length 123), which extends from the proximal hub 112 to adjacent the distal tip 140, , as shown in Fig. 1A. In the example shown in Fig. 2A, the inner diameter at the distal tip 140 tapers down along internal taper T1 from ID1 to a relatively smaller inner diameter ID2. In one such embodiment, the taper length for internal taper T1 is equal to about 0.22" (cm 0.56) and ID2 extends for a distance S1 for about 0.100" (0.254 cm) and ID2 has a value equal to about 0.056" (0.142 cm). In some examples, the inner diameter then further transitions from ID2 along an internal taper T2 to an even smaller inner diameter ID3. In some embodiments, the distal portion of the distal tip (length S2) has a length from about 0.71 cm to about 0.74 cm, and in some more specific embodiments, a length from about 0.721 cm to about 0.726 cm. In a specific instance, taper T2 extends for a distance equal to about 0.044" (0.112 cm), where the ID3 is equal to about 0.034" (0.086 cm) and extends for a length S2 of about 0.285" (0.724 cm). In some alternative embodiments, S1 is equal to zero, whereby internal taper T1 and internal taper T2 are adjacent to each other to thereby provide a smooth transition of internal diameter. Some alternative embodiments include the dilator shaft substantially comprising a low density polyethylene or a polyether ether ketone, with some such embodiments of the dilator shaft having a hardness from about 40 shore D to about 85 shore D.

Some embodiments of the dilator shaft comprised of a relatively harder material (e.g. HDPE) have an inner diameter ID1 of about 0.072 inches (0.18 cm) to about 0.11 inches (0.28 cm). Other embodiments of the dilator shaft comprised of a relatively softer material (e.g. polyurethanes, polyether block amide) have an inner diameter ID1 of about 0.050 inches (0.13 cm) to about 0.11 inches (0.28 cm). Polyether block amide (PEBA) is a thermoplastic elastomer (TPE) and is known under the tradenames of VESTAMID^{®} E (Evonik Industries) and Pebax^{®} (Arkema).

In the example shown in Fig. 2A, having several internal transitions, such as internal taper T1 and internal taper T2, ensures that the hybrid dilator has an OD along its proximal length (OD1) that enables the hybrid dilator 100 to dilate a tissue puncture site to a desired extent, while at the same time allowing the wall thickness Wₚ of the distal tubing 121 to be maintained to provide shaft rigidity and stiffness that is comparable to a conventional sheath/dilator assembly. The internal geometry of distal tip 140, including dual tapers T1 and T2 and the inner diameter along the distal tip 140, provides for insertion of a puncturing device such as needle there-through and for the desired extension of a needle tip. The internal geometry also helps ensure that the wall thickness W_{Tip} (Fig. 2B) at the distal edge 148 of the distal tip 140 is sufficiently thin to ensure crossing and trackability through the transseptal puncture site. Still furthermore, the dual tapers T1 and T2 ensure that a smooth transition is provided between the relatively wider inner diameter ID1 along the proximal portion of distal tubing 121, and the relatively narrower inner diameter ID3 at the distal edge 148. In some embodiments, the inner diameter ID3 at distal edge 148 is about 0.033 inches (0.084 cm) to about 0.037 inches (0.094 cm) and the outer diameter at distal edge 148 is about 0.040 inches (0.10 cm) to about 0.055 inches (0.14 cm). In one specific example, the inner diameter ID3 at the distal edge 148 is equal to about 0.034" (0.086 cm) (Fig. 2B) and the outer diameter OD2 at the distal edge is equal to about 0.042" (0.107 cm).

In some embodiments, the taper angle TA may range from about 5° to about 15°. In some examples, the taper length of external taper T3 may range from about 1.0 cm to about 1.6 cm. In some embodiments, length of the external taper T3 ranges from about 0.4 inches (1 cm) to about 1 inch (2.5 cm). In one example, the taper length of external taper T3 may be about 1.0 cm with a taper angle TA of about 15°. In some embodiments, the wall thickness W_{Tip} at the distal edge 148 of the distal tip 140 is between about 4 thousandths of an inch (0.010 cm) to about 5 thousandths of an inch (0.013 cm). The wall thickness W_{Tip} is sufficient for maintaining mechanical integrity of the distal tip 140 while ensuring that it is not too thick to make it difficult for the distal tip 140 to cross a puncture site within the tissue.

In an alternative embodiment of the present disclosure, as shown in Fig. 2C, a distal tip 140 may be provided with a single internal taper T1 as shown. As shown, the distal tubing 121 is shown with inner lumen visible.

### Wall Thickness, Bending Stiffness and Torque

As discussed earlier with respect to Fig. 2A, the hybrid dilator 100 is an HDPE Dilator with a 12.5 French OD with an 8.5 French ID. The ID and OD are representative of the dimensions along the proximal length 123 of the distal tubing 121. Additionally, the wall thickness Wₚ along the proximal length 123 is about 25.5 thousandths of an inch (0.065 cm) to about 27.5 thousandths of an inch (0.070 cm). Bending stiffness for the illustrated example is about 3 N/mm and the torque is about 4.5 N cm.

In an alternative embodiment the hybrid dilator is a 12.5 French OD dilator with an 8.5 French ID. The wall thickness Wp along the proximal length 123 of the distal tubing 121 is about 32 thousandths of an inch (0.081 cm). Bending stiffness for the particular example is about 4 N/mm and the torque is about 5 N cm.

In still a further alternative, the hybrid dilator 100 is a 12.5 French OD dilator with a 4.5 French ID. The wall thickness Wp along the proximal length 123 of the distal tubing 121 is about 55 thousandths of an inch (0.140 cm). Bending stiffness for the particular example is about 5.5 N/mm and the torque is about 7 N cm. In another example, the hybrid dilator is a 15 French dilator where the wall thickness is less than about 26.5 thousandths of an inch (0.067 cm) to provide adequate stiffness.

In some embodiments, a HDPE hybrid dilator 100 has: a 12.5 French OD which is about 0.162-0.166" (0.411-0.422 cm); a 4.5-8.5 French ID (about 0.056-0.115 inches or about 0.142-0.292 cm); a wall thickness from about 0.025" to about 0.055" (about 0.064-0.140 cm), a stiffness of about 3.5 to 5.5 N/mm, and a torque transmission from about 4 to about 7 N cm.

In an alternative embodiment, the dilator shaft is comprised substantially of HDPE and includes: a 12.5 French OD (about 0.162"-0.166" or about 0.411 - 0.422 cm); an 8.5 French ID (about 0.108"-0.115" or about 0.274-0.2921 cm); a wall thickness from about 23.5 thousandths of an inch (0.06 cm) to about 29 thousandths of an inch (0.074 cm). Such embodiments may have a bending stiffness from about 2.5 to 3.5 N/mm and a torque transmission from about 4 to 4.5 N cm.

In another alternative embodiment, the dilator shaft is HDPE and has: a 12.5 French OD (about 0.162"-0.166" or about 0.411-0.422 cm); a 7.5 French ID (about 0.095"-0.102" or about 0.241-0.259 cm); and a wall thickness which is about 0.03-0.036" (about 0.076-0.091 cm). Bending stiffness for such examples is about 3.5 to 4.5 N/mm and the torque transmission is about 4.5 to 5.5 N cm. In some specific embodiments, the wall thickness is about 32 thousandths of an inch (0.081 cm).

Another alternative embodiment includes the dilator shaft being comprised of HDPE and the shaft having: a 12.5 French OD (about 0.162"-0.166" or about 0.411-0.422 cm); a 4.5 French ID (about 0.056"-0.063" or about 0.142-0.160 cm); and a wall thickness of about 0.05-0.055" (0.127-0.140 cm). Typically, bending stiffness for such embodiments is from about 5 to 6 N/mm and the torque is about 6 N cm to 7 N cm. In some specific embodiments, the wall thickness is about 55 thousandths of an inch (0.140 cm).

In an alternate embodiment, the dilator shaft has an outer diameter between 12 French (about 0.162"-0.166" or about 0.411-0.422 cm) and 18 French (about 0.236" or about 0.599cm). In some embodiments, the inner diameter is configured to accommodate a needle such a mechanical needle or an RF needle. In some alternate embodiments, the inner diameter is configured to accommodate a wire such as an RF wire. Additionally, the inner diameter of the dilator shaft may be modified to adjust the mechanical properties of the dilator shaft. For example, a dilator shaft with an outer diameter of 18 French may have an enlarged inner diameter to reduce the wall thickness of the dilator shaft and thereby reduce the stiffness. In another embodiment, the material of the dilator shaft is adjusted to achieve the desired mechanical properties.

In some embodiments of the present disclosure Torque may range from about 1.0 N cm to about 7 N cm over a length of about 50 cm. In some examples the bending stiffness ranges from about 1.0 N/mm. to about 5.5 N/mm over a span of 50 mm.

### Surface Finish

In some embodiments of the present disclosure, the distal tubing 121 may comprise different surface finishes to provide various amounts of friction along the exterior surface. In some embodiments, as above the distal tubing 121 may be formed substantially of HDPE. Alternatively, the dilator may be formed from multiple material layers or a composite material. In some such examples, the multiple layers may extend concentrically and longitudinally along the length of the distal tubing 121 in the form of multiple tubular layers. In one such example the inner layer or tubing comprises an HDPE or a low density polyethylene (LDPE) core with an outer layer of Pebax (polyether block amide) extrusion. This may provide a relatively smoother exterior finish compared to HDPE. Furthermore, the Pebax tubing allows for silicone coating to be disposed thereon to additionally provide a smooth coating on the exterior.

### Alternate embodiments of the distal tip

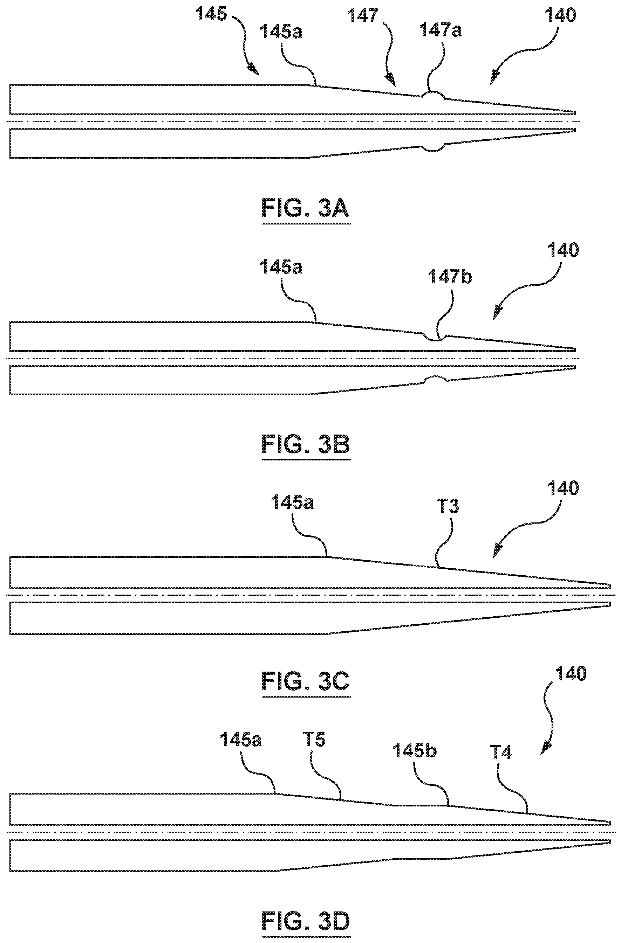

In an alternate embodiment of the present disclosure, as shown in Figs. 3A to 3D, the distal tip 140 comprises a modified taper. In one specific example as shown in Figs. 3A and 3B, the tapered distal tip 140 may comprise a secondary feature such as a secondary surface modification 147 that creates a surface variation, such as a secondary bump 147a or a divot 147b to more closely create the tactile queues of a standard sheath/dilator transseptal kit. The first tactile cue comes from a first/primary feature such as a first surface modification 145, which may be a first bump 145a that is represented by the transition between the tapered tip 140 and the proximal length 123 of the distal tubing 121. As above, the second tactile cue comes from the secondary surface modification 147, for example the secondary bump 147a or divot 147b.

Alternatively, as shown in Fig. 3C, the tapered distal tip 140 may comprise a smooth single external taper T3 with a single surface modification such as a first surface modification145 in the form of a first bump 145a at the transition, as described previously. In a further alternative, there may be two or more external tapers along the exterior. In a specific example, the distal tip 140 may have two external tapers: external taper T4 and external taper T5 as shown in Fig. 3D, where the first surface modification 145 and secondary surface modification 147 are formed by transitions that form first bump 145a and second bump 145b. These provide tactile cues during use as the hybrid dilator 100 is being advanced through, for example, the septum. The tactile cues mimic the cues that are generally obtained from transitions in a standard transseptal kit that includes a standard dilator and sheath assembly while still providing a smooth transition such that the device does not get stuck at the tissue boundary. In some such examples, the internal taper may be as shown in Fig. 2A comprising internal tapers T1 and T2.

### Alternatives

In alternative embodiments of the present disclosure, the distal tip 140 may have a modified external taper T3. In some such examples, the geometry of the external taper T3 may be varied. As outlined previously, the distal tip 140 may have surface modifications along the external taper T3. The external taper T3 may be provided with a secondary bump 147a, the external taper T3 may be provided with divot 147b. Alternatively, the external taper T3 may be provided with a modified roughness.

In alternative embodiments, the ID of the distal tip 140, including internal taper(s), is modified in order to accommodate a crossing/puncturing device such as a needle (for example an RF needle). Alternatively, internal geometry may be modified in order to accommodate a crossing/puncturing device such as a guide wire (for example an RF guidewire). In some embodiments, the shaft distal tubing 121 comprises a single material. Alternatively, the shaft distal tubing 121 may comprise a composite material via co-extrusion or post extrusion processing/layering. In some examples, the shaft distal tubing 121 comprises a lubricious coating material along the exterior. In some such examples, the chemistry and/or processing of the lubricious coating material is varied to provide a suitable coating. In some embodiments, material may be used within the distal tubing 121, and for coating, in accordance with what is known in the art. In a further alternative of the present disclosure, the hybrid dilator 100 may be provided with forward facing ports along, the distal tip 140, to allow for fluid injection when a needle or a guidewire is positioned inside the hybrid dilator 100.

In some embodiments of the present disclosure the hybrid dilator 100 has been created to optimize the tubing stiffness/torque response. Also, the handle/hub 112 provides enhanced handing features (discussed further herein below). In some embodiments, as shown previously, the distal tip 140 is provided with two external distal tapers. In some embodiments, the internal controlled geometry may be provided in varying configurations.

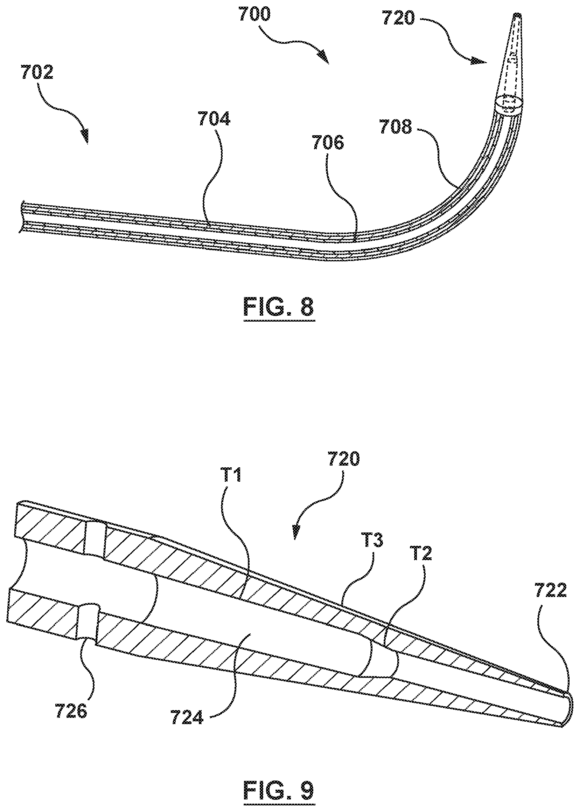

Fig. 8 is a cross sectional view of the shaft and distal tip of a hybrid dilator of an alternative embodiment of the present disclosure and Fig. 9 is an enlarged view of the distal tip of Fig 8, wherein the dilator shaft has more than one layer and the tip is typically comprised of the same material as one of the shaft layers.

Hybrid dilator 700 of Fig. 8 has a shaft 702 which includes three layers, inner layer 706, outer layer 708, and a middle layer, torque layer 704, to improve the torqueability of the device. There is a smooth joint between device tip 720 and a shaft 702. Inner layer 706 is typically comprised of HDPE and outer layer 708 typically of Pebax or LDPE. Typical embodiments of shaft 702 provide a mechanical response that is similar to transseptal sheath and dilator sets that physicians commonly currently use. The durometer of the Pebax may be selected to adjust the flexibility and pushability of the shaft. The torque layer is typically a braided material, while in alternative embodiments the torque layer may be a stiff polymer and/or a metallic hypotube. Some further embodiments of shaft 702 do not include torque layer 704. While outer layer 708 is typically comprised of Pebax or LDPE, in some alternative embodiments it is made of HDPE or other density blends of polyethylene that achieve the desired properties of flexibility and torque, all of which are compatible with lubricious coatings. Typical embodiments of shaft 702 have an outer diameter at least the size of current transseptal sheaths (approximately 0.144" (0.366 cm)) to dilate the septum to at least the same size as current sheaths, and have a mechanical response (including flexibility, pushability, and torqueability) comparable to current transseptal sheath and dilator pairings. Some embodiments of shaft 702 have a 12.5 F outer diameter of about 0.163 "(0.414 cm) to about 0.166" (0.421 cm). Other embodiments of shaft 702 have a 15 F outer diameter of about 0.193" (0.490 cm) to about 0.205" (0.521 cm). Some embodiments of shaft 702 which have the torque layer 704 have a torque transmission from about 4 N cm to about 8 N cm, with one specific embodiment having a torque transmission of about 8.1 N cm.

In embodiments which include a torque layer 704 between the inner and outer materials (For example HDPE and Pebax), the braid normally functions as an anchor between the inner and outer layers. Such embodiments may be manufactured using a reflow process which melts both the inner and outer layers into the braided layer whereby the braided layer mechanically joins the two materials together. Some such embodiments have a stainless steel braid and provide 8 N cm of torque transmission.

Fig. 9 illustrates an embodiment of tip 720 typically comprised of HDPE with from about 20 percent to 50 percent of the distal tip being comprised of BaSO4 to facilitate imaging, but alternatively may be comprised of Pebax or any thermoplastic. In some embodiments, tip 720 is comprised of about 40% BaSO4. In testing, HDPE has displayed the advantageous characteristic of being stiff enough to be skive resistant. Tip 720 of Fig. 9 includes internal lumen 724, distal edge 722, and a single external taper T3 for smooth dilation. Internal taper T1 and internal taper T2 guide devices (e.g. guidewires, needles) from the shaft into the tip area, and limits needle protrusion (of compatible needles) out of the end of the dilator. The illustrated example includes two distal side holes 726 for limiting vacuum and pressure formation when withdrawing devices, while alternative examples include different size, location, number of holes, and configuration of holes. In other embodiments an alternate radiopacifier is embedded in the polymer material such as BiOCL. In some embodiments, the BiOCL is <25%. In another embodiment, a change in radiopaque materials is used to visualize the distal tip 1004. Other embodiments of tip 720 include radiopaque features such as bands and coils made from radiopaque materials (e.g. platinum, gold, tungsten, and/or barium sulfate-filled polymer).

Making further reference to Fig. 9, the inner diameter of tip 720 varies from the shaft ID to a smaller diameter compatible with commonly used 0.032" (0.081 cm) or 0.035" (0.089 cm) devices (e.g. guidewires and needles). The length of the external taper T3 is typically more than 1.0 cm long since a shorter length increases the crossing force or may make crossing tissue more abrupt, with some examples of tip 720 having a taper length T3 up to 3 cm in length. In some embodiments, the external taper length of the external taper T3 ranges from about 0.4" (1 cm) to about 1" (2.5 cm). The outer diameter of tip 720 is typically no greater than 0.055" (0.140 cm) or else the force in advancing through tissue would be larger than typical transseptal dilators. As an example, if the device is 0.032" (0.081 cm) compatible and has an ID of approximately 0.034" (0.086 cm), restraining the tip OD to a maximum of 0.054" (0.137 cm) facilitates smooth advancement through tissue.

In a specific embodiment of the hybrid dilator 700 shown in Figures 8 and 9, shaft 702 has an outer diameter of 0.164 inches (0.417 cm) and an inner diameter of 0.072 inches (0.183 cm), the inner diameter of tip 720 at distal edge 722 is compatible with device having outer diameters of 0.032 inches (0.081 cm) or 0.035 inches (0.089 cm), the maximum tip OD is less than 0.055 inches (0.140 cm), the two side holes 726 have diameters of about 0.012 inches (0.030 cm) to about 0.024 inches (0.061 cm), and external taper T3 has a length of 1.6 cm. Typical dilators have a taper length of approximately 1 cm and a smaller diameter than the illustrated embodiment. To prevent hybrid dilator 700 from having a higher taper angle than typical dilators (which results in a higher crossing force), hybrid dilator has an external taper T3 with a length of 1.6 cm which corresponds with its relatively larger outer diameter. In some embodiments, the inner diameter of tip 720 at distal edge 722 is about 0.033 inches (0.084 cm) to about 0.037 inches (0.094 cm) and the outer diameter of tip 720 at distal edge 722 is about 0.040 inches (0.10 cm) to about 0.055 inches (0.14 cm).

Further alternative embodiments of hybrid dilator 700 include outer layer 708 of shaft 702 being made of thermoplastic to facilitate manufacturing. Some examples have only one internal lumen taper or more than two. Some further embodiments include an electrode configured for puncturing at the tip so that the one device can puncture, cross, and dilate.

Some embodiments include the shaft having an inner layer 706 made of HDPE and an outer layer 708 made of Pebax, wherein, during manufacture of the device, tip 720 and inner layer 706 are formed in the same extrusion of HDPE whereby tip 720 and inner layer 706 are continuous without any internal joint, which eliminates the risk of a sharp needle being advanced through the dilator catching at a joint between the dilator shaft 702 and tip 720.

### Proximal hub

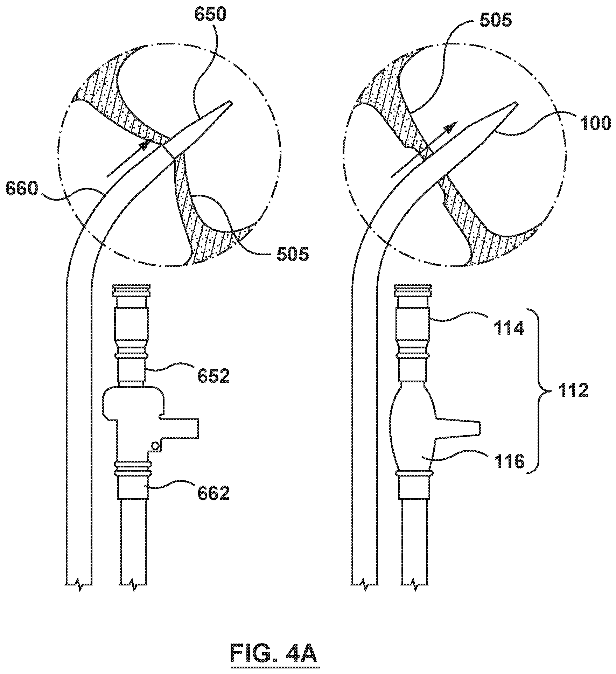

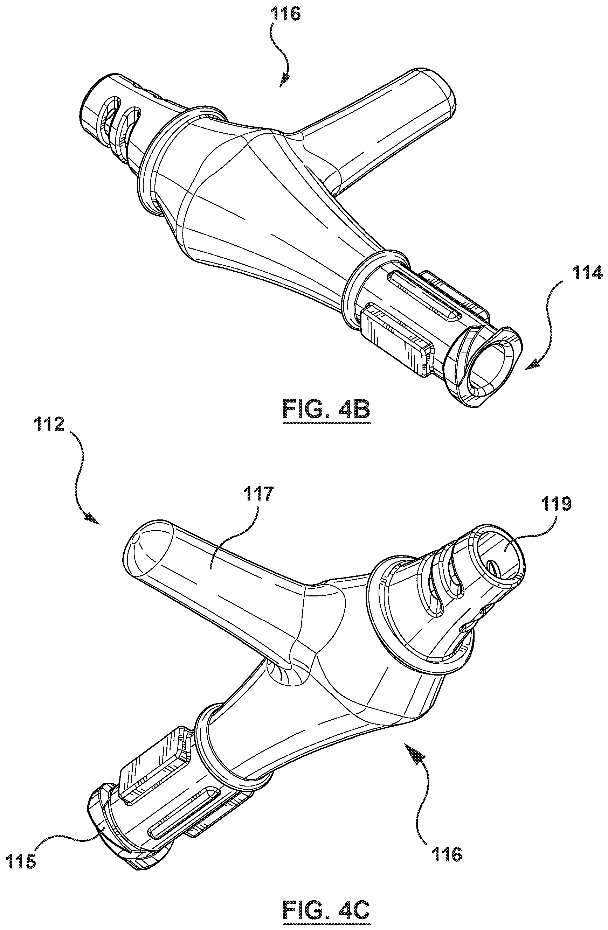

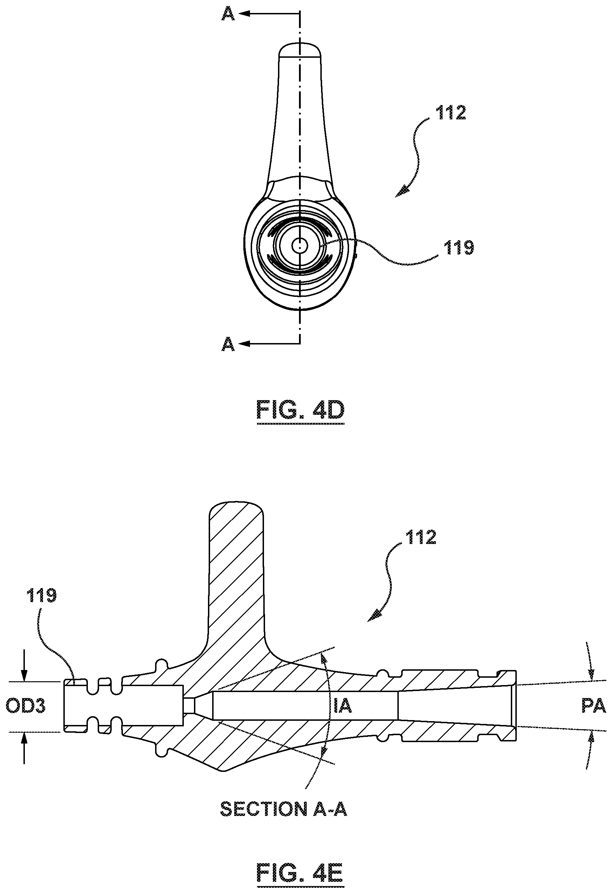

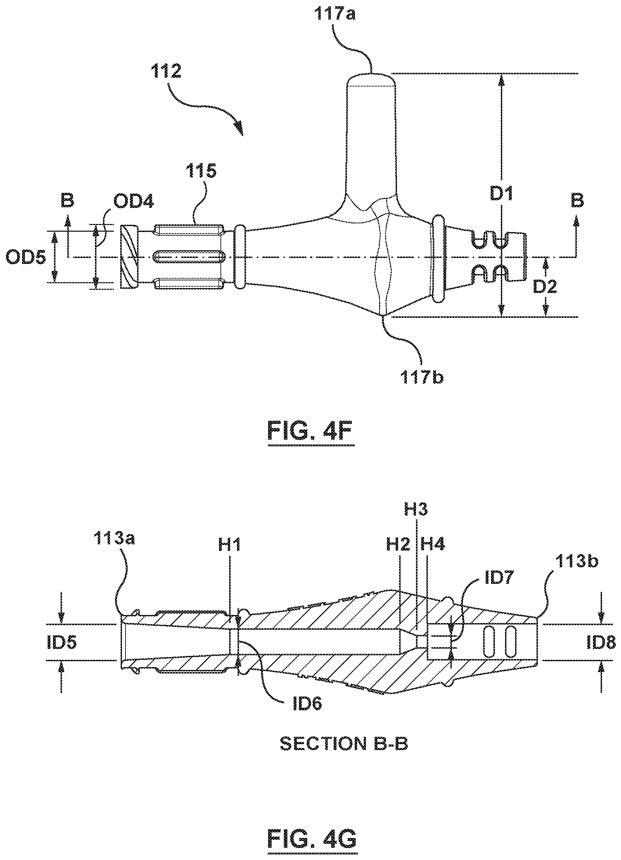

The hybrid dilator 100 comprises a handle defined by a hybrid or combination proximal hub 112 at a proximal end thereof, as additionally shown in Fig. 4A. The proximal hub 112 comprises dilator hub 114 that is formed integrally with a sheath hub or a sheath-like hub 116. Fig. 4A also includes a prior art dilator 650 inserted into sheath 660 such as to show a dilator hub 652 and a sheath hub 662 proximally, and dilator 650 extending out of sheath 660 distally. Sheath 660 and dilator 650 are being advanced across a septum 505 but the heart tissue is catching on sheath 660. In contrast, hybrid dilator 100 which is being advanced across septum 505 without snagging. In some embodiments as shown in Figs 4B-4C and 4F, the dilator hub 114 comprises a Luer hub or Luer connector 115 and the sheath hub 116 comprises an arm 117 that functions as pseudo side-port that provides the functional feel of a side-port to provide an indication/direction of the distal end curvature. The arm 117 mimics the side-port of a standard sheath without providing the fluid capability of a standard sheath side-port. The proximal hub 112 forms a hub/handle that is larger than a standard transseptal dilator hub so as to provide the physician with similar handling and expected tactile feedback, by featuring additional material to hold onto and additionally provides the arm 117 to indicate the direction of the distal end curvature. In some examples, the arm 117 may be replaced by functional side-port if the fluid capability is desired. In one specific example, the proximal hub 112 comprises a custom insert molded HDPE Hub at the proximal end with a luer connector 115 and tactile features (defined by a side-port arm 117) to indicate the plane of distal curvature and provide similar handling characteristics. In some such examples, the proximal end 110 has a luer taper to allow for connection of medical syringes or fluid drips. Fig. 4D illustrates an end view taken from a distal end of the proximal hub 112 showing a coupling 119 of the proximal hub 112 for connecting the proximal hub 112 to the distal tubing 121. In some such examples the coupling 119 may comprise a strain relief. Figs. 4E and 4G show cross-sectional views of the proximal hub 112 illustrating the internal configuration of the proximal hub 112, which may include features for facilitating entry of other devices therein during use. In some such examples, the proximal hub 112 comprises HDPE.

Proximal hub 112, as illustrated in Fig. 4E, includes an outer diameter OD3 of 5.25 mm at its distal end, an internal angle IA of 40.0 degrees, and a proximal angle PA of 6.0 degrees. Proximal hub 112, as illustrated in Fig. 4F, the proximal column has an outer diameter OD5 of 6 mm and an outer diameter of OD4 of 7.37 mm at the Luer connector at its proximal end. The distance D1 between arm endpoint 117a and opposing point 117b is 28.39 mm, and the distance D2 between opposing point 117b and the central longitudinal axis of proximal hub 112 is 6.49 mm. Proximal hub 112, as illustrated in Fig. 4g, has an inner diameter ID5 of 4.25 mm internal to the hub proximal end 113a, an inner diameter ID6 of 3 mm at the innermost portion of the lumen, an inner diameter ID7 at the narrowest portion of the lumen, and an inner diameter ID8 of 4.12 mm at the hub distal end 113b of the hub. Other hub dimensions shown in Fig. 4G include: hub location H1 (at the distal end of the proximal internal taper) is 12.40 mm from hub proximal end 113a, hub location H2 (at the proximal end of the distal internal taper) is 31.83 mm from hub proximal end 113a, hub location H3 (at the distal end of the distal internal taper) is 33.75 mm from hub proximal end 113a, and hub location H4 (at the distal end of narrowest portion of the lumen) is 35 mm from hub proximal end 113a.

### Alternate embodiments of the proximal hub

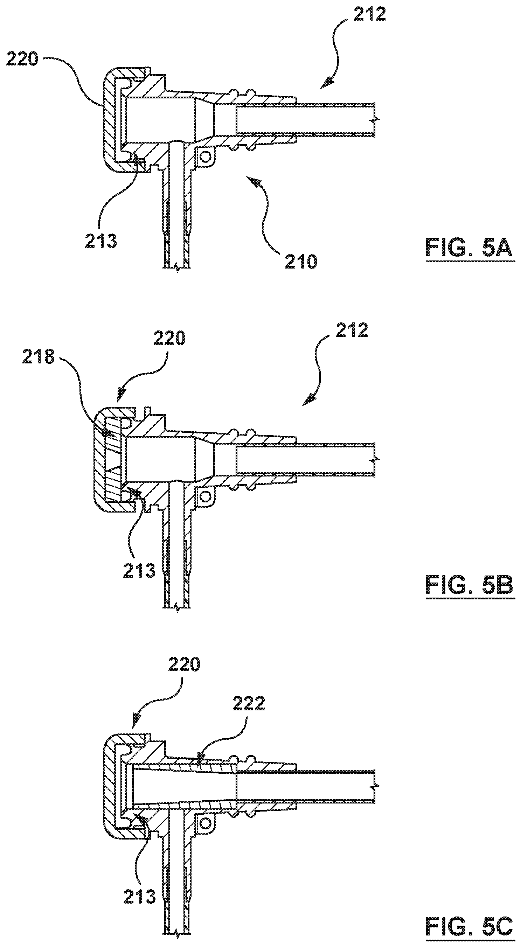

In some embodiments as shown in Figs. 5A-5D, an alternate embodiment of a hybrid dilator 200 is provided with a modified proximal portion 210. The hybrid dilator 200 comprises a valved proximal hub 212, as shown in Figs. 5A-5B, where the hub comprises a valve 213 at its proximal end with a cap 220 for retaining the valve in position. The valve 213 is provided as a hemostasis valve. In some examples, as shown in Fig. 5B, the valved proximal hub 212 may additionally comprise an extra feature to direct devices into the valve 213. In some embodiments the proximal hub 212 has an insertion guide 218 as a molded or an external feature that function co-operatively with the valve to direct and align product being inserted into the valve 213. In the particular example shown, the insertion guide 218 is provided proximal of the valve 213.

In accordance with another embodiment of the present disclosure, a feature is provided within the valved proximal hub 212 to funnel device into the shaft tubing. In a particular case, a funnel guide 222 is provided to direct and align product inserted into valve 213 into the shaft tubing. The funnel guide is positioned distal of the valve 213. In some such examples, the funnel guide 222 is provided as a molded feature. In some embodiments, funnel guide 222 is configured such that it also centers the proximal end of the guidewire with respect to the valve. This centering directs the proximal end of the guidewire when it is inserted through the device's distal tip for the purpose of device exchange.

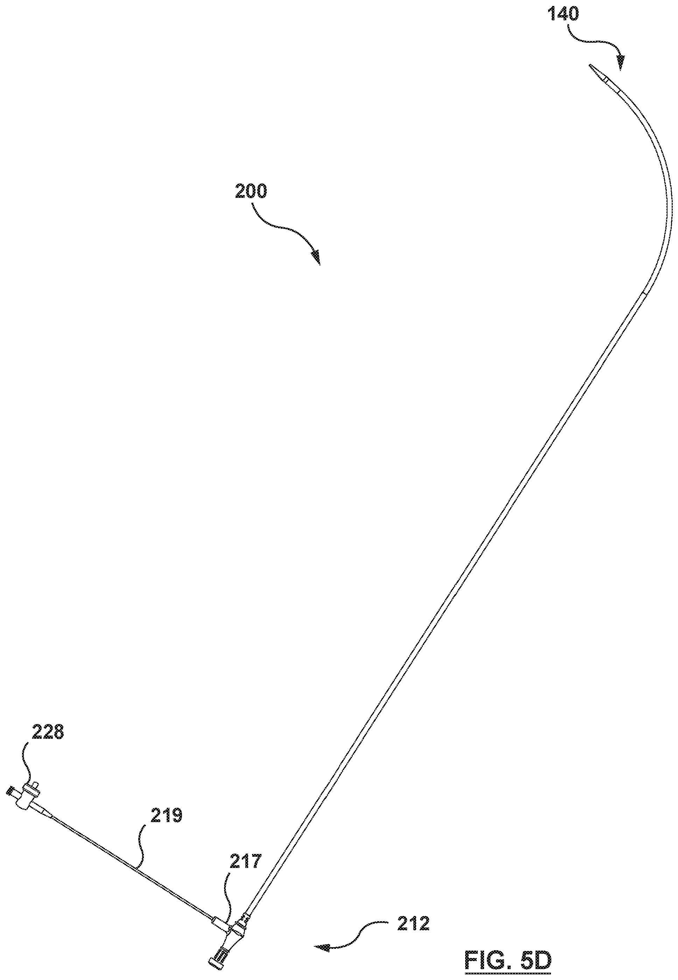

In a further alternative, as shown in Fig. 5D, a hybrid dilator 200, is provided with a proximal hub 212 that houses a valve 213, for example a hemostasis valve, and additionally comprises a side-port port 217 that has a side-port tubing 219 attached thereto, with a stopcock 228 to provide for flushing and aspiration.

In alternate embodiments of the present disclosure, the proximal hub 212 may comprise material that is taken from the group consisting of Pebax, HDPE, LDPE, and Nylon or a combination thereof to achieve desired lubricity and handling characteristics.

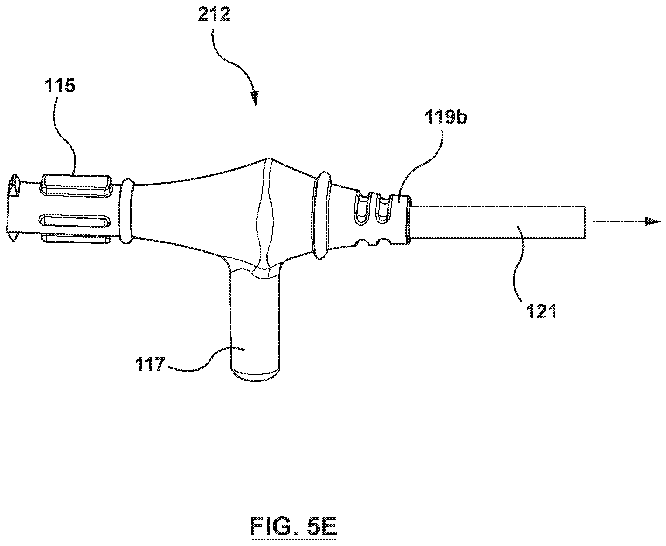

In still a further alternative, a proximal hub 112 is shown in Fig. 5E, that comprises a Luer connector 115 according to ISO 594-1,-2. Additionally, an arm 117 is provided in the form of a mock side-port to provide expected handling and align with the distal curve. Additionally, the proximal hub 112 is provided with a strain relief 119b at its distal end and the distal tubing 121 extends in a distal direction out of strain relief 119b.

### Alternatives

In some embodiments of the present disclosure, the proximal hub 112 or valved proximal hub 212 may comprise a molded hub. In some embodiments, the proximal hub 112 or valved proximal hub 212 may comprise HDPE. Alternatively, other materials may be used. In some embodiments, the geometry of the hub may be varied as may be suitable. In alternative embodiments of the valved proximal hub 212, the valve material and/or geometry may be varied as may be known in the art. In some such examples, the slit configuration and/or size may be varied to provide a suitable valve to meet the requirements of the procedure, such as a transseptal procedure. In still further alternatives, the material of the side-port tubing, and the ID and OD of side-port tubing may be selected and/or varied as may be known to a person skilled in the art. Similarly, in some examples, as shown in Fig. 5D where a stopcock is provided, the stopcock material may be varied as may be known in the art.

In still a further alternative of the present disclosure, some embodiments of a hybrid dilator of the present disclosure may provide the simplicity of transseptal crossing, and yet may still allow an ablation catheter to be used with it in case the need arises.

Another aspect of the disclosure is a kit for puncturing a tissue comprising: a crossing device having a puncturing feature; and a hybrid dilator 100, wherein the dilator has a dilator shaft defining a lumen 122 for receiving the crossing device therethrough, the dilator shaft being structured to provide support for the crossing device when the crossing device is used to create a puncture in a tissue. The hybrid dilator also includes a distal tip 140 having an outer diameter which substantially tapers down to an outer diameter of the crossing device for cooperatively providing a smooth profile when the hybrid dilator 100 is advanced through a tissue over the crossing device. In some embodiments of the kit, the crossing device is a mechanical needle with a sharp tip, while in some other embodiments, the crossing device is configured for delivering energy to a tissue.

Another aspect of the disclosure is a system for puncturing a tissue comprising: a crossing device having a puncturing feature which is operable to deliver energy to a tissue; an electrosurgical generator which is operable to provide energy to the puncturing feature; and a hybrid dilator 100, wherein the hybrid dilator has a dilator shaft defining a lumen 122 for receiving the crossing device therethrough, the dilator shaft being structured to provide support for the crossing device when the crossing device is used to create a puncture in a tissue. The hybrid dilator also includes a distal tip 140 having an outer diameter which substantially tapers down to an outer diameter of the crossing device for cooperatively providing a smooth profile when the hybrid dilator is advanced through a tissue over the crossing device.

### Methods of performing a transseptal procedure using a hybrid dilator, guidewire and crossing device

In accordance with the present disclosure, a method of the present disclosure provides for streamlining the procedural workflow by providing a hybrid dilator that combines the functionalities of a conventional transseptal sheath and dilator assembly. With the hybrid dilator of the present invention a reduced number of devices may be required in order to complete the transseptal procedure, which enhances procedural efficiency while reducing procedural time and complexity.

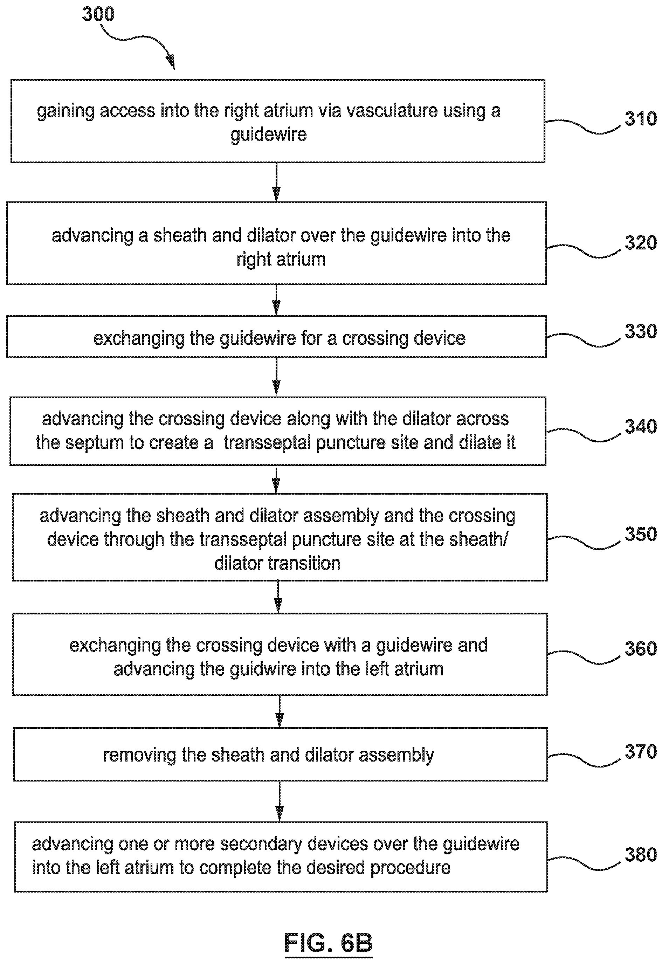

In such example, a method of the present disclosure avoids the disadvantages associated with a conventional transseptal procedure. Figs. 6A and 6B illustrate an example of method of performing such a conventional transseptal medical procedure 300. The method comprises the steps of: at step 310, gaining access into the right atrium 501 via vasculature using a guidewire; at step 320, advancing a sheath 20 and dilator 40 over the guidewire into the right atrium 501, the sheath 20 and dilator 40 forming a sheath and dilator assembly 50; at step 330, exchanging the guidewire for a crossing device 60 which comprises a puncturing device 62; at step 340, advancing the crossing device 60 along with the dilator across a septum 505 to create a transseptal puncture site 510 and dilate the transseptal puncture site. At step 340, the sheath 20 may get hung up at the sheath/dilator interface and the transition between the sheath/dilator can affect a physician' s ability to cross tissue in a predictable, repeatable fashion. Sometimes the physician may not able to cross through to the sheath (get the sheath across the septal puncture site because the tissue will get hung up at the sheath/dilator interface). If at step 350, the physician is successful, the physician may be able to advance the sheath and dilator assembly 50 and the crossing device 60 through the transseptal puncture site 510 to enable the sheath and dilator transition to cross the puncture site 510. In some such procedures, the physician may wish to use a relatively large delivery sheath (for example which is larger than the transseptal sheath 20) for complex procedures for example for cryoablation procedures or a left atrial appendage closure/occlusion procedure and knows they cannot cross with the large delivery sheath, so the physician will introduce a standard transseptal kit with the sheath and dilator as discussed at step 350 above to purely to cross and pre-dilate the septum. Once this three piece kit is removed for exchange, it must be disposed, thereby underutilizing the three items (sheath, dilator, guidewire) for only a short procedural presence. The removal of the sheath/dilator assembly and exchange with the larger delivery sheath is described further below. At step 360 of the method, the crossing device 60 is exchanged with a guidewire 80, which comprises the steps of removing the crossing device 60 and advancing the guidewire 80 into the left atrium 502; at step 370, removing the sheath and dilator assembly 50; and at step 380, advancing one or more secondary devices 70 such as a relatively large delivery sheath over the guidewire 80 into the left atrium 502 to complete the desired procedure.

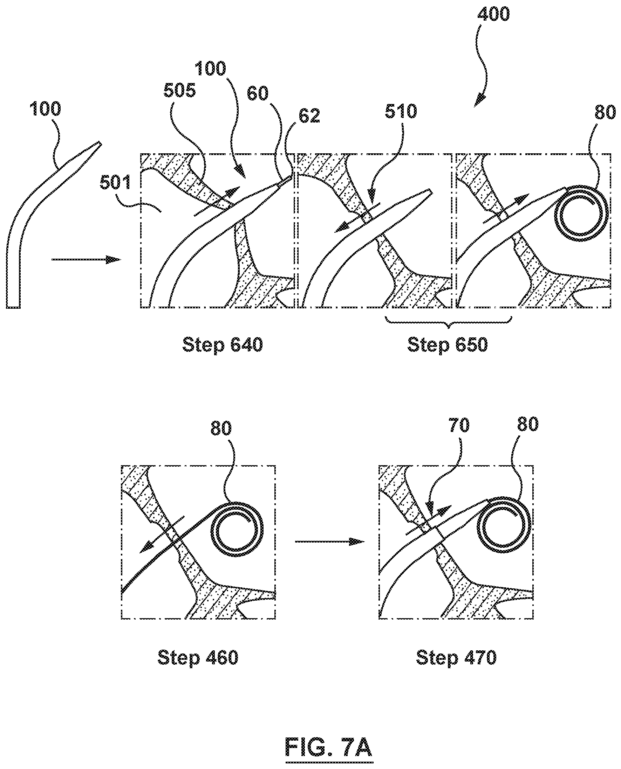

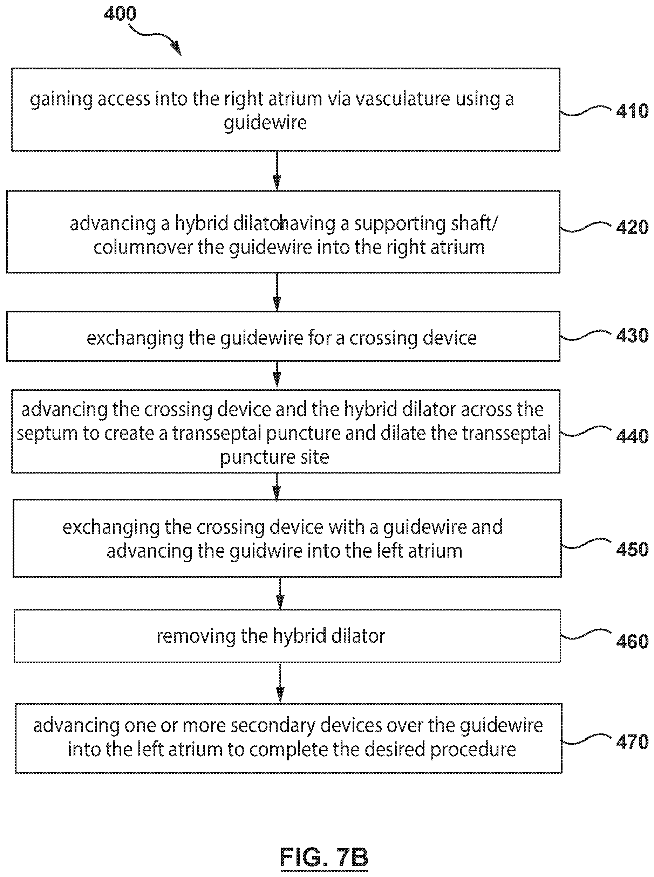

As outlined herein above, embodiments of the present disclosure provide an optimized transseptal procedure In accordance with a method of the present disclosure, as shown in Figs. 7A and 7B, an optimized method 400 is provided for carrying out a transseptal procedure. The method comprises the steps of: at step 410, gaining access into the right atrium via vasculature using a guidewire; and at step 420, advancing a hybrid dilator 100 having a supporting shaft/column over the guidewire into the right atrium 501; By using the hybrid dilator 100 it reduces number of parts that the physician is required to prep/assemble and introduce into the patient from three to two. Instead of a sheath, dilator and guidewire, a hybrid dilator 100 and guidewire may be used. The method additionally provides: at step 430, exchanging the guidewire for a crossing device 60 which comprises a puncturing device 62 [In some embodiments of the present disclosure, the puncturing device 62 may comprise a needle. In some such examples, the needle is a radiofrequency (RF) needle. Alternatively the needle may comprise a mechanical needle. In other embodiments of the present disclosure, the puncturing device 62 may comprise a radiofrequency (RF) guidewire]; and at step 440 advancing the crossing device and the hybrid dilator across the septum 505 to create a transseptal puncture site 510 and dilate the puncture site 510 to facilitate advancement of one or more secondary devices 70 through the transseptal puncture site. The hybrid dilator 100, which may also be referred to as the step-up dilator, is provided as a simplified tool. It simplifies the procedural workflow by providing a one piece transseptal tool compared to a sheath and dilator (it is additionally usable with a guidewire and needle as shown). The hybrid dilator 100 is provided as a one/single oversized dilator and in use it reduces the number of physical/geometric transitions as well as the number of material transitions or tactile obstructions which may allow the physicians to complete a transseptal or other tissue crossings with greater ease. The hybrid dilator 100 reduces the changes of the hybrid dilator 100 from getting caught at the transseptal puncture site, by provided smooth lines and tapers to facilitate a seamless transition across tissue. This allows the hybrid dilator 100 to be advanced across the septum with greater ease. The method additionally provides for; at step 450, exchanging the crossing device 60 with a guidewire 80 and advancing the guidewire 80 into the left atrium; at step 360, removing the hybrid dilator 100; and at step 470, advancing the one or more secondary devices over the guidewire 80 into the left atrium 502 to complete the desired procedure.

In procedures where the physician wishes to use a relatively large delivery sheath for complex procedures (for example for cryoablation or LAA occlusion) and knows they cannot cross with that product, the physician can now introduce just the hybrid dilator 100 over a guidewire as discussed in step 420 (as depicted in Fig. 7B) using a single device to cross and pre-dilate the septum. The hybrid dilator 100 and the initial guidewire may then be removed for exchange, thus using only two products (hybrid dilator 100 and guidewire, instead of a standard sheath, dilator and guidewire kit). As such the improved method additionally provides at steps 460 and 470 removing just the hybrid dilator 100 to allow exchange with the secondary device such as a relatively large delivery sheath for complex procedures, wasting fewer products in the process.

Another embodiment of the method uses a hybrid dilator 100 and a crossing device for puncturing a septum 505 of a heart. This embodiment of the method comprises the steps of: a) positioning a distal tip 140 of the hybrid dilator at a desired site of the septum; b) using the hybrid dilator 100 for supporting a crossing device, located within a lumen of the hybrid dilator, as the crossing device is advanced beyond the distal tip of the hybrid dilator to puncture the septum; and c) advancing the hybrid dilator over the crossing device thereby dilating the desired site. In some such embodiments, the crossing device is a mechanical needle and step (b) further includes applying force with the mechanical needle to the septum to thereby puncture the septum. In other embodiments, the crossing device is configured for delivering energy, and step (b) further includes supplying electrical energy to the crossing device to thereby puncture the septum. Some embodiments further comprise a step (d) of exchanging the crossing device with a guidewire and advancing the guidewire into a left atrium, a step (e) of removing the hybrid dilator, and a step (f) of advancing one or more secondary devices over the guidewire into the left atrium.

In some embodiments of using a hybrid dilator and a crossing device for puncturing a septum of a heart wherein the crossing device is configured for delivering energy, the crossing device is further configured for use as a guide-wire, and the method further comprises a step (d) of removing the hybrid dilator, and typically, a step (e) of advancing one or more secondary devices over the crossing device into a left atrium. Further details of crossing devices suitable for delivering energy and using as a guide-wire are given in International Publication No. WO2015019132, entitled "METHODS AND DEVICES FOR PUNCTURING TISSUE", which is hereby cited.

### Hybrid Dilator and Method of Use

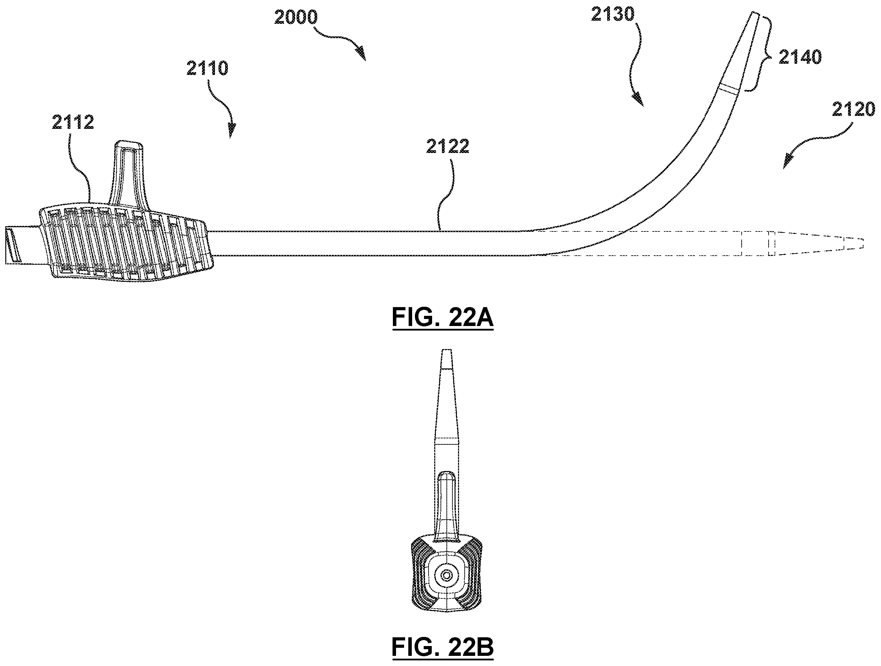

In accordance with an embodiment of the present disclosure, a hybrid dilator 2000 is shown in Figs. 22A - 22D. The hybrid dilator 2000 comprises a combination of features that provide a dual functionality of a sheath and a dilator for facilitating a transseptal puncture procedure while avoiding disadvantages of conventional sheath and dilator assemblies. The hybrid dilator 2000 functions as a single device that removes the need for using a conventional sheath/dilator assembly resulting in less waste and a simplified workflow. The hybrid dilator 2000 comprises a sheath-like handle with familiar torque and tactile control. In the specific example shown, the hybrid dilator 2000 defines a proximal portion 2110 comprising a molded combination proximal hub 2112 as shown in Fig. 22A. A distal portion 2120 is coupled to the proximal portion 2110 comprising a dilator shaft. The dilator shaft extends from the proximal end and defines a curved distal end 2130 that terminates in a distal tip 2140. The hybrid dilator 2000 comprises a lumen 2122 there-through that narrows at the distal tip 2140.

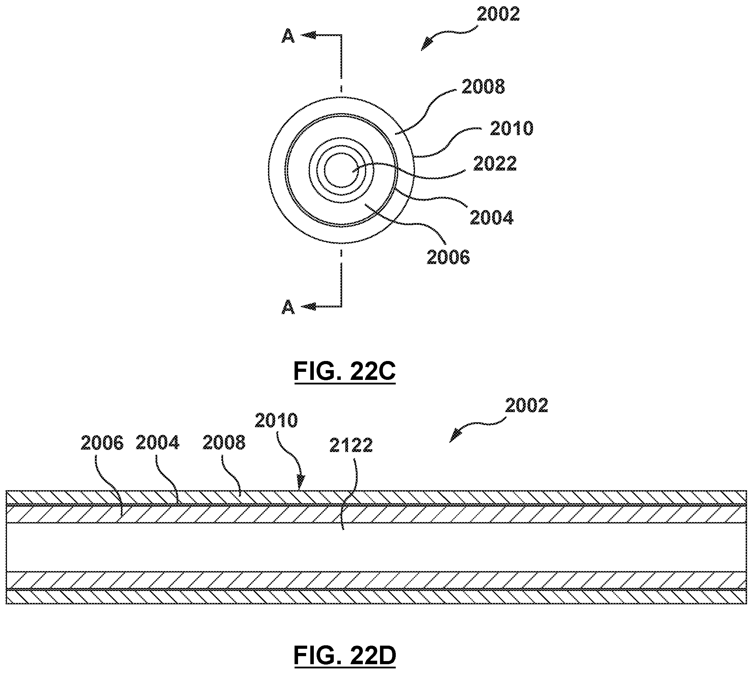

The dilator shaft is shown in Figs. 22C and 22D. The hybrid dilator 2000 is a single unitary device and the dilator shaft provides mechanical properties to best facilitate procedural activities. The hybrid dilator 2000 is sufficiently rigid to enable positioning and advancement of a crossing device, such as a puncturing needle or wire, within the hybrid dilator 2000 while maintaining the position of the assembly at a desired site. As such, the hybrid dilator 2000 functions to provide support and columnar strength to facilitate placement of the crossing device at the desired location.

As shown in Fig. 22A, in some embodiments of the present disclosure, the distal end 2130 of the hybrid dilator 2000 may be curved. Alternatively, the distal end 2130 of the hybrid dilator 2000 may be straight. The curved distal end 2130 facilitates advancement of the hybrid dilator 2000 in conjunction with the puncturing device to initiate a transseptal puncture.

Fig. 22D shows a cut-away view of the hybrid dilator's 2000 shaft 2002. The hybrid dilator 2000 has a shaft 2002 which includes three layers, an inner layer 2006, an outer layer 2008, and a middle torque layer 2004. The torque layer 2004 improves the torquability of the device. The inner layer 2006 is comprised of HDPE and the outer layer 2008 is comprised of LDPE. The torque layer 2004 is a braided material comprised of stainless steel. The braid functions as an anchor between the inner and outer layers. Such embodiments may be manufactured using a reflow process which melts both the inner and outer layers into the braided layer whereby the braided layer mechanically joins the two materials together. Some embodiments of shaft 2002 having torque layer 2006 have a torque transmission from about 2 N cm to about 8 N cm. In some specific embodiments, the torque transmission is from about 2 N cm to about 6 N cm. In one specific embodiment, the shaft 2002 has a torque transmission of about 4 N cm. In an alternate embodiment, the shaft 2002 has a torque transmission of about 8 N cm. In some embodiments, an outer coating 2010 is disposed on the outer surface 2008 to provide a smooth coating on the exterior. In one embodiment, the outer coating 2010 is a silicone coating.

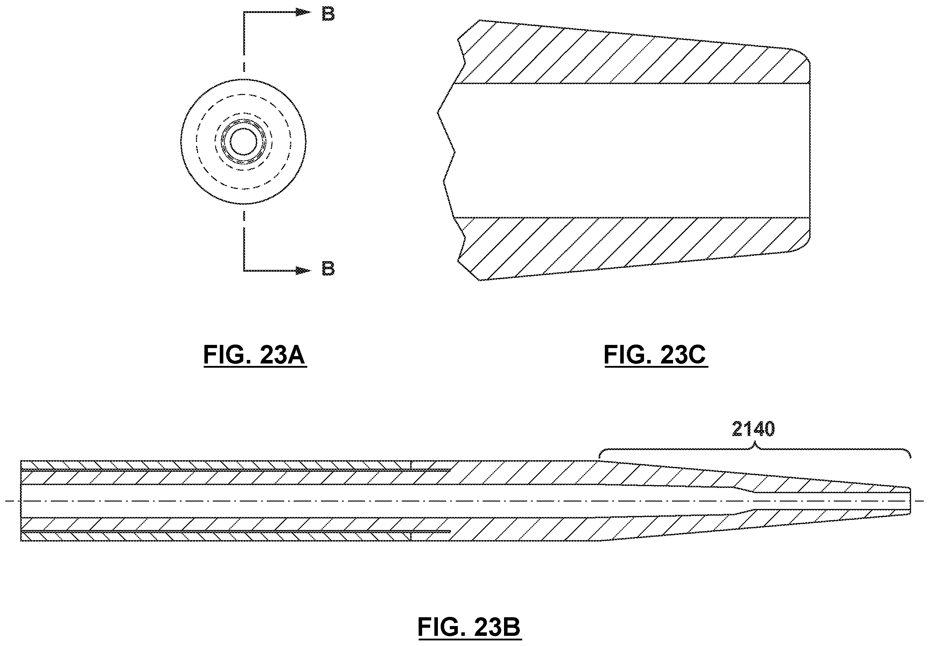

Hybrid dilator 2000 comprises a smooth joint between the shaft 2002 and the device tip 2140. The distal tip 2140 of the hybrid dilator 2000, as illustrated in Fig. 23B, transitions through a smooth external taper. The taper allows dilation of the septum to an appropriate size for the subsequent delivery device or equipment that may be used. The outer diameter of the hybrid dilator 2000 is substantially constant from the proximal edge of distal tip 2140 to the proximal hub 2112. In some such embodiments, the outer diameter of the hybrid dilator 100 may vary based on the application and clinical use. In some embodiments, the size of hybrid dilator 2000 is from about 12 French to about 20 French. In a specific example, the hybrid dilator has the size about 12.5 French (outer diameter of about 0.163" (0.414 cm) to about 0.166" (0.421 cm)). In the embodiment of Fig. 23B, the taper of the distal tip 2140 has an external taper length of 0.63" (1.6 cm). The outer diameter of the distal end of the tapered distal tip 2140 is a dimeter of 0.055" (0.14 cm).

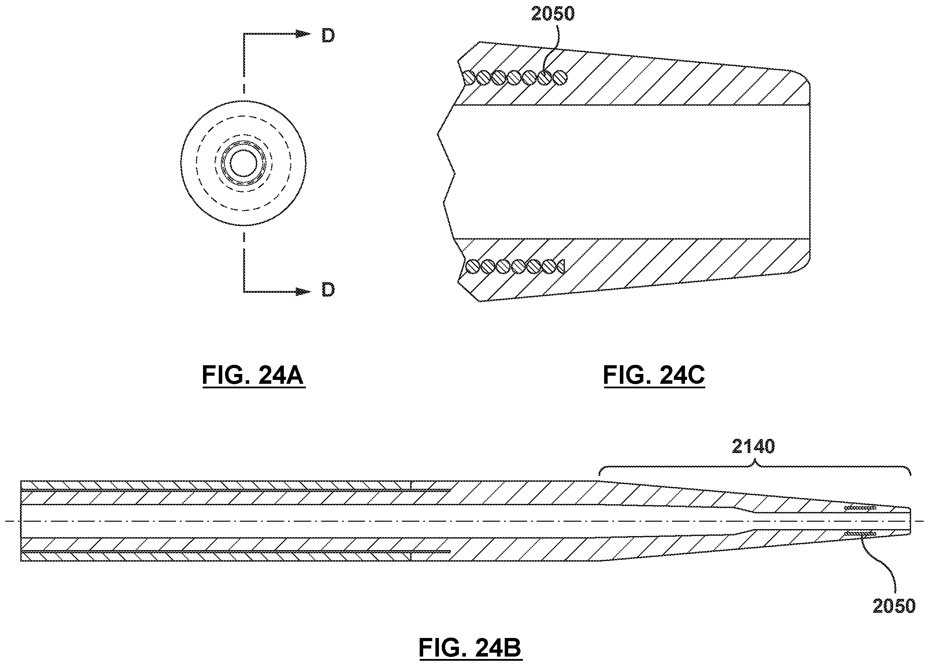

Figs. 24A - 24C show an alternate embodiment of the distal tip 2140 of the hybrid dilator 2000. In this embodiment, the distal tip 2140 comprises a radiopaque marker 2050. In the embodiment of Fig. 24 B, the radiopaque marker 2050 is a marker coil. In addition to being radiopaque, the marker coil 2050 is an echogenic marker and is visible under ultrasound. In one embodiment, the marker coil is from about 0.04" (1 mm) to about 0.08" (2 mm) in length. In a specific embodiment, the marker coil is about 0.063" (1.6 mm) in length, has a 0.053" (1.35 mm) outer diameter and a 0.004" (0.1 mm) coil diameter. In some embodiments, the marker coil is placed from about 0.04" (1 mm) to about 0.1" (2.5 mm) from the distal edge of the distal tip 2140. The distance between the distal edge and the radiopaque marker 2050 provide optimal visibility under ultrasound or fluoroscopy and maintain visibility of the crossing device's distal tip while inside the hybrid dilator 2000. In some embodiments, the distal tip 2140 comprises an additional radiopacifier embedded in the polymer material such as BiOCL. In one embodiment, the distal tip 2140 is comprised of 15% BiOCL.

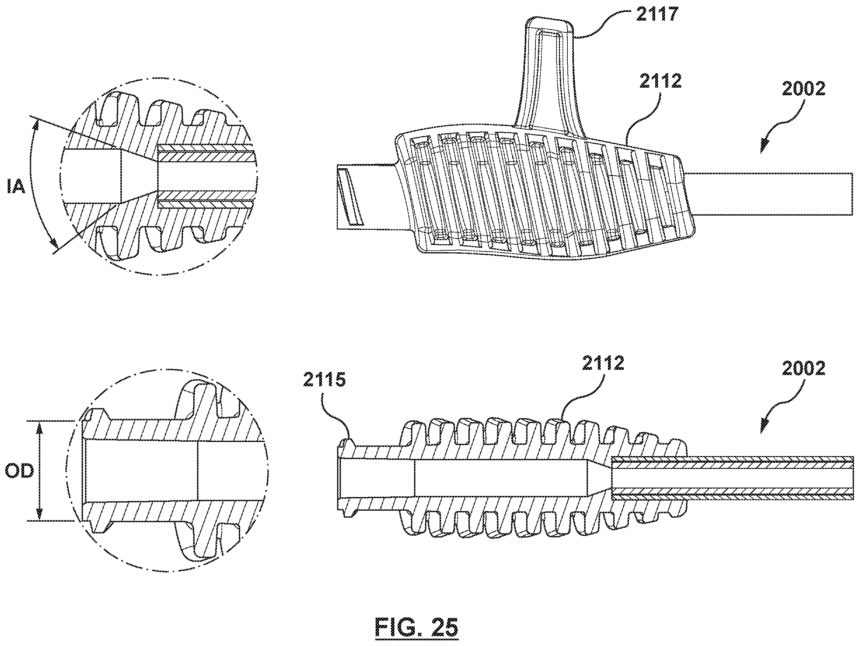

The hub 2112 of the hybrid dilator 2000 is shown in Fig. 25. The hub 2112 comprises a Luer hub or Luer connector 2115 and an arm 2117 to provide an indication/direction of the distal end curvature. The proximal hub 112 forms a hub/handle that is larger than a standard transseptal dilator hub so as to provide the physician with similar handling and expected tactile feedback of the combination of a dilator hub and a sheath hub, by featuring additional material to hold onto and additionally provides the arm 117 to indicate the direction of the distal end curvature. In a specific example, the proximal hub 2112 comprises a custom insert molded HDPE Hub at the proximal end with a Luer connector 2115 and arm 2117 to indicate the place of distal curvature. In some such examples, the proximal end has a Luer taper to allow for connection of medical syringes, pressure measurements, or fluid drips. The hub 2112 as illustrated in Fig. 25 comprises and outer diameter of 6.123mm at the Luer connector at its proximal end. The proximal hub 2112 has an internal angle IA of 40.0 degrees.

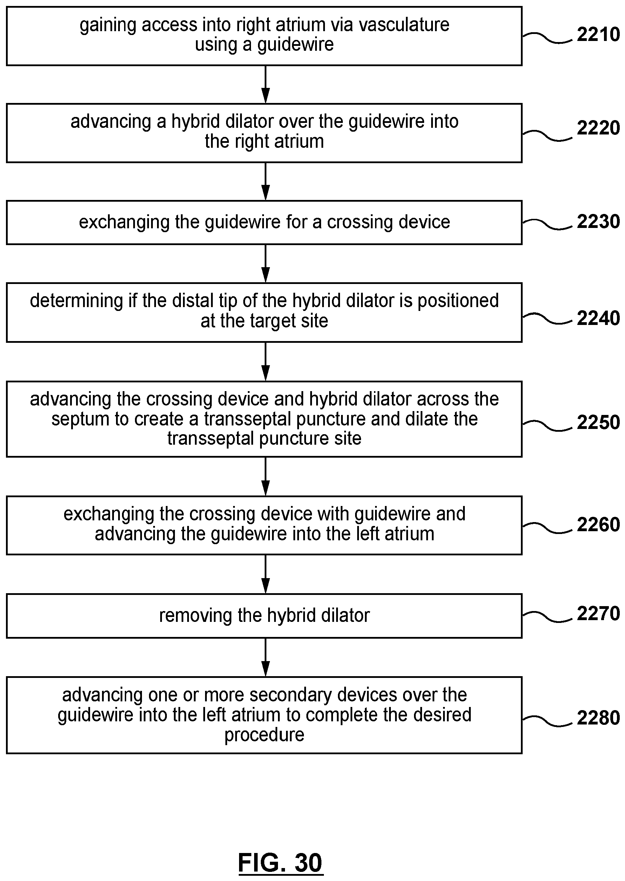

An example of an optimized transseptal workflow with the use of the hybrid dilator 2000 is illustrated in Fig. 30. The method comprises the steps of: at step 2210, gaining access into the right atrium via vasculature using a guidewire; and at step 2220, advancing a hybrid dilator 2000 over the guidewire into the right atrium. By using the hybrid dilator 2000, it reduces the number of parts that the physician is required to prep/assemble and introduce into the patient. Instead of a sheath, dilator and guidewire, a hybrid dilator 2000 and guidewire may be used. This eliminates the need to separately exchange the sheath and dilator. The method additionally provides: at step 2230, exchanging the guidewire for a crossing device such as a mechanical needle or an RF needle and step 2240 determining if the distal tip of the hybrid dilator is positioned on the target site. The position of the distal tip 2140 of the hybrid dilator 2000 may be determined using various visualization methods such as fluoroscopy, electro-anatomical mapping (EAM), or echogenic markers. The hybrid dilator 2000 may be repositioned until it is in the correct position. Once in the correct position, the step 2250 comprises advancing the crossing device and the hybrid dilator across the septum to create a transseptal puncture site and dilate the puncture site to facilitate advancement of one or more secondary devices through the transseptal puncture site. Due to the crossing device and the hybrid dilator respectively having at least one echogenic, EAM, or radiopaque marker, the relative positioning of the distal tip of the dilator and the distal tip of the crossing device may be seen at all times. By observing when the distal tip of the crossing device advances past the distal tip of the hybrid dilator through one of the visualization methods, the physician will be able to determine when the crossing device has crossed into the left atrium. The hybrid dilator 2000 simplifies the procedural workflow by providing a one-piece transseptal tool compared to a sheath and dilator. The hybrid dilator 2000 reduces the number of physical/geometric transitions as well as the number of material transitions or tactile obstructions which may allow the physicians to complete a transseptal crossing with greater ease. The method may additionally comprise step 2260, exchanging the crossing device with a guidewire and advancing the guidewire into the left atrium; at step 2270, removing the hybrid dilator 2000, and at step 2280, advancing the one or more secondary devices over the guidewire into the left atrium to complete the desire procedure.

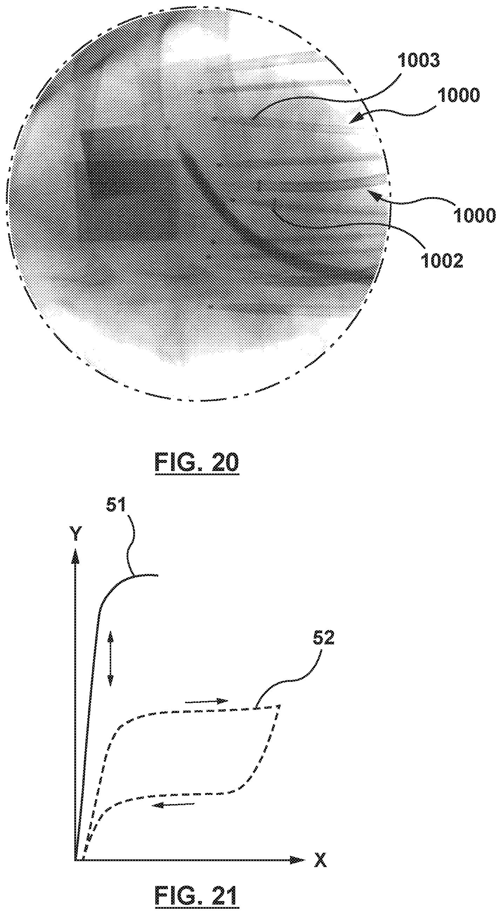

The radiopaque marker 2050 allows visualization of the distal tip 2140 of the hybrid dilator 2000 (See Fig. 20). The visualization allows the user to determine the position of the hybrid dilator's tip 2140 relative to the target position. This allows the user to adjust the hybrid dilator's position prior to using the crossing device. Once the distal tip 2140 of the hybrid dilator 2000 is correctly positioned, the crossing device may be used to cross the target tissue. In some embodiments, the crossing device that is provided comprises one or more radiopaque markers at a distal end thereof. In some such embodiments, the one or more crossing device radiopaque markers 12 are configured to co-operate with the hybrid dilator radiopaque marker 2050 to indicate the position of the crossing device relative to the hybrid dilator. A successful puncture and crossing by the crossing device may be determined by the radiopaque markers of the crossing device moving relative to the radiopaque marker 2050 of the hybrid dilator 2000. Once the crossing device has crossed the target tissue, the hybrid dilator 2000 is advanced. The position of the distal tip 2140 of the hybrid dilator 2000 may be tracked during dilation and after crossing the target tissue.

In some embodiments, the distal tip of the hybrid dilator 2000 comprises a material with radiopacifier properties (such as Bismuth oxychloride (BiOCL)) embedded in the polymer material, the distal tip 2140 is visible under fluoroscopy (See Fig. 20). Both the radiopaque marker of the crossing device at the distal tip of the hybrid dilator are visible, even when the crossing device is positioned within the hybrid dilator 2000. In other words, the radiopaque marker 2050 and the radiopacifier embedded polymer distal tip 2140 are visible on fluoroscopy and ultrasound. When the crossing device is positioned within the hybrid dilator, the marker band of the crossing deice is more opaque than the distal tip 2140 of the hybrid dilator allowing both devices to be visible. In this embodiment, when the distal tip 2140 of the hybrid dilator 2000 is positioned against the target tissue, a successful puncture may be determined either by the visualization of the crossing device exiting the distal tip or the visualization of the crossing device under ultrasound.

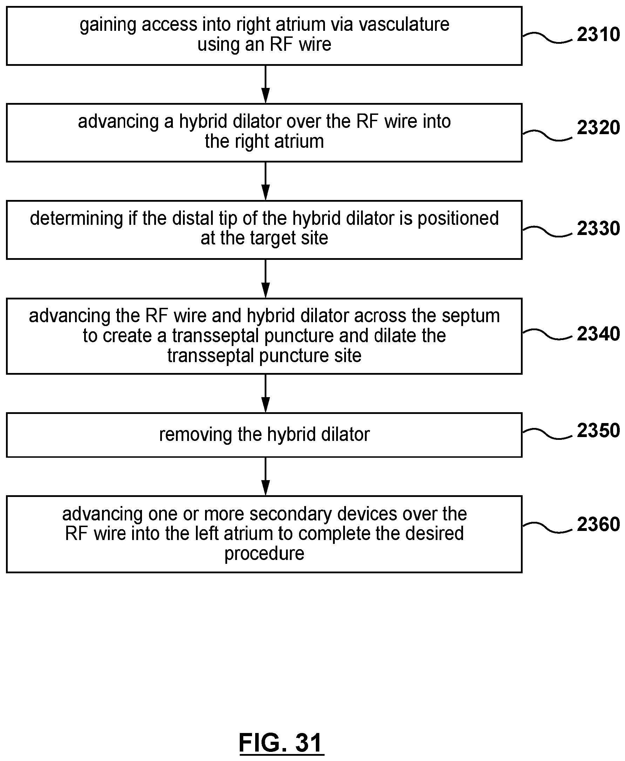

An alternate example of an optimized transseptal workflow with the use of the hybrid dilator 2000 is illustrated in Fig. 31. The method comprises the steps of: at step 2310, gaining access into the right atrium via vasculature using a radiofrequency (RF) wire; and at step 2220, advancing a hybrid dilator 2000 over the RF wire into the right atrium. By using an RF wire and a hybrid dilator 2000, it reduces the number or parts that the physician uses. Instead of a sheath, dilator, guidewire, and needle, a hybrid dilator and RF wire may be used. The method may additionally comprise: step 2320, determining if the distal tip of the hybrid dilator is positioned on the target site. The position of the distal tip 2140 of the hybrid dilator 2000 may be determined using various visualization methods such as fluoroscopy, electro-anatomical mapping, or echogenic markers. The hybrid dilator 2000 may be repositioned until it is in the correct position. Once in the correct position, step 2340 comprises advancing the RF wire and the hybrid dilator across the septum to create a transseptal puncture site and dilate the puncture site to facilitate advancement of one or more secondary devices through the transseptal puncture site. The hybrid dilator 2000 simplifies the procedural workflow by providing a one-piece transseptal tool compared to a sheath and dilator. The hybrid dilator 2000 reduces the number of physical/geometric transitions as well as the number of material transitions or tactile obstructions which may allow the physicians to complete a transseptal crossing with greater ease. The method may additionally comprise step 23, removing the hybrid dilator 2000 while the RF wire remains in the left atrium, and at step 2360, advancing the one or more secondary devices over the RF wire into the left atrium to complete the desire procedure.

### Reshapeable Hybrid Dilator

During a transseptal puncture procedure, the hybrid dilator is positioned against the septum 505. Proper position may be verified using visualizing systems such as fluoroscopy and intracardiac echocardiography (ICE). In some embodiments, the hybrid dilator is visualized on an electroanatomical mapping (EAM) system. Once in proper position at the target site, the crossing device punctures the septum 505.

In some instances, the hybrid dilator is unable to reach the target site due to anatomical variations. In standard workflows, the crossing device is a rigid mechanical needle or rigid RF needle that may be manipulated or curved to shape to the patient's anatomy. Accordingly, the shape of the crossing device is typically designed to define the curve of the combined system (i.e., hybrid dilator and crossing device) so that the tip of the crossing device may be appropriately directed.

In some instances, the rigid needle is unable to be shaped or curved to the desired shape or the desired shape is not maintained after shaping or curving. In other embodiments, the crossing device is a flexible puncturing device and does not impart any rigid shape or curve to the overall system at all.

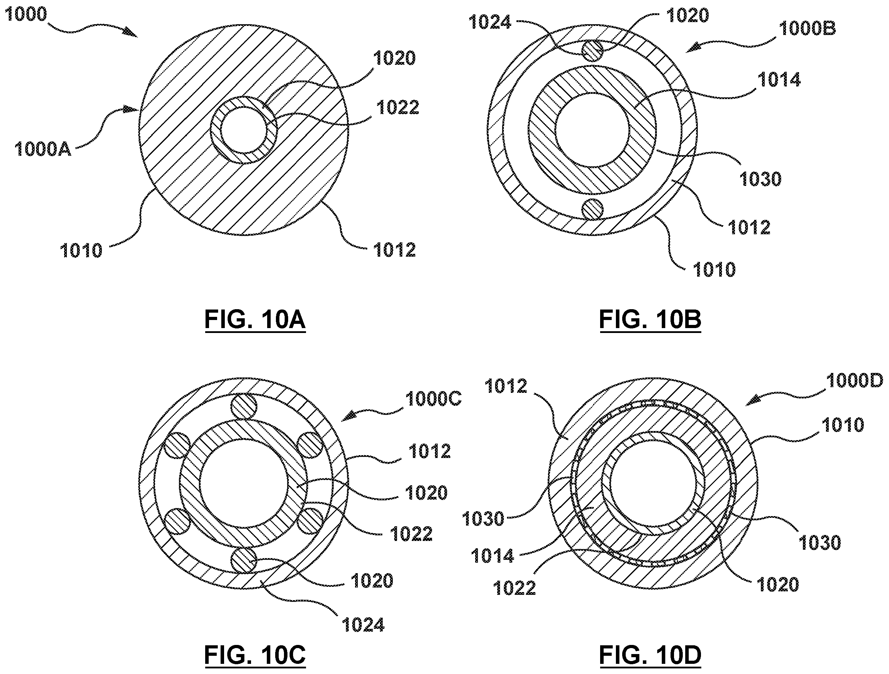

In an embodiment, the hybrid dilator can be manipulated or curved to the shape of the patient's anatomy. In a specific embodiment, the hybrid dilator 1000 is reshapeable. A reshapeable hybrid dilator 1000 allows for enough plasticity to deform to the desirable shape, and enough rigidity to resist relaxation of the curve during device manipulation in the body. Figures 10A- 10D show a cross sections of various embodiments of a reshapeable hybrid dilator 1000. Hybrid dilator 1000 comprises a stiffening member 1020 which provides the physician the ability to re-shape the dilator during the procedure, optimizing the positioning of the distal tip on the septum.

The shapeability of the hybrid dilator 1000 allows physicians to shape the hybrid dilator 1000 to improve positioning on the septum while also providing increased reach of the distal tip (i.e. increased distal tip distance from an uncurved axis). If the physician is not satisfied with the positioning of the tip of the hybrid dilator, the physician may shape the hybrid dilator 1000 to a desired curvature. This allows the physician to shape or curve the hybrid dilator 1000 so the tip of the hybrid dilator has the desired lateral distance from the axis of the uncurved portion. The stiffening member 1020 enables the hybrid dilator to be shaped either prior to or during the procedure. The stiffening member 1020 may be plastically deformed by manual manipulation by a physician. Once hybrid dilator 1000 has been manipulated to a desired curvature, the stiffening member 1020 has sufficient rigidity to resist relaxation of the curve. In other words, the stiffening member 1020 enables the hybrid dilator 1000 to hold its shape throughout the procedure, or until the hybrid dilator 1000 is again manipulated to a difference shape. When navigated through the vasculature to the target site, the stiffening member 1020 holds the desired curve of the hybrid dilator 1000.

The hybrid dilator 1000A of Fig. 10A has a shaft 1010 comprising an outer layer 1012 and a stiffening member 1020. In this embodiment, the stiffening member 1020 is a hypotube 1022. The outer layer 1012 is typically comprised of Pebax or LDPE. In some alternative embodiments, the outer layer is made of HDPE or medium density polyethylene (MDPE), all of which are compatible with lubricious coatings. In some embodiments, the stiffening member 1020 is a stiff homogenous core such as a hypotube 1022 made of metal or plastic. In some embodiments, the metal hypotube may undergo heat treatments or other conditioning to achieve the desired properties. In alternate embodiments, the stiffening member 1020 is a stiff heterogenous core formed of multiple layers and segments of metal and/or plastic.

The hybrid dilator 1000B of Fig. 10B has a shaft 1010 comprising an outer layer 1012 and an inner layer 1014. The outer layer 1012 is typically comprised of Pebax or LDPE. The inner layer 1014 is typically comprised of HDPE. The hybrid dilator 1000B comprises a stiffening member 1020 between the inner layer 1014 and the outer layer 1012. In the embodiment of Fig. 10B, the stiffening member 1020 is a wire stiffener 1024. The wire stiffener 1024 comprises at least one wire. In the embodiment shown, two wires are present. In some embodiments, the wire stiffener is made of metal and in alternate embodiments, the wire stiffener is made of a polymer. In some embodiments, the wire stiffeners undergo conditioning or treatments to achieve desired properties. In the embodiment of Fig. 10B, the hybrid dilator 1000B additionally comprises a torque layer 1030. The torque layer 1030 improves the torqueability of the device. Torqueability refers to the ability of a device to respond to manual manipulations at one end of the device (e.g., a proximal end) to translate to movement at a second end of the device (e.g., a distal end). The torque layer is typically a braided material. The torque layer 1030 is positioned between the inner and outer layers 1012, 1014 with the wire stiffeners 1024. In some embodiments, the outer layer 1012 and the inner layer 1014 are fixed to one another. Such embodiments may be manufactured using a reflow process which melts both the inner and outer layers into the braided layer whereby the braided layer mechanically joins the two materials together. In other embodiments, the outer layer 1012 and the inner layer 1014 are bonded to one another (e.g. adhesives, friction fit, etc.). In some embodiments, the outer layer 1012 and inner layer 1014 are fixed to one another along the length of the shaft. In some alternate embodiments, the outer layer 1012 and inner layer 1014 are fixed to one another at intervals along the shaft. The layers may be fixed at intervals to alter the mechanical properties along the shaft 1010 to improve steering or shapeability considerations.

The hybrid dilator 1000C of Fig. 10C has a shaft 1010 comprising an outer layer 1012 and multiple stiffening members 1020. The hybrid dilator 1000C comprises a first stiffening member, wire stiffeners 1024, and a second stiffening member, hypotube 1022. The combination of stiffening members results in a combination of reshapeable properties. The hypotube may be a homogenous core or a heterogenous core. The hybrid dilator 1000C comprises at least one wire stiffener 1024. In this specific embodiment, hybrid dilator 1000C has 6 wire stiffeners 1024.

The hybrid dilator 1000D of Fig. 10D has a shaft 1010 comprising an outer layer 1012, an inner layer 1014, and a stiffening member 1020. In this embodiment, the stiffening member 1020 is a hypotube 1022. In this specific embodiment, the stiffening member 1020 is the innermost layer and defines the lumen. The hybrid dilator 1000D additionally comprises a torque layer 1030. The torque layer 1030 is typically a braided material. The torque layer 1030 is positioned between the inner and outer layers 1012, 1014. Such embodiments may be manufactured using a reflow process which melts both the inner and outer layers into the braided layer whereby the braided layer mechanically joins the two materials together. Some such embodiments have a stainless steel braid and provide 8 N cm of torque transmission.

In some embodiments, the size of hybrid dilator 1000 is from about 12 French to about 20 French. In a specific example, the hybrid dilator has a size of about 12.5 French (outer diameter of about 0.163" (0.414 cm) to about 0.166" (0.421 cm)). In another example, the hybrid dilator has a size of about 15 French (outer diameter of about 0.193" (0.490 cm) to about 0.205" (0.521 cm)).

### Hybrid Dilator Superelastic Curve Retention

During a transseptal puncture procedure, the hybrid dilator is tracked through the vasculature to reach the heart. In cases of difficult percutaneous access or tracking through complex vasculature, the hybrid dilator may be deformed and lose the desired curve. A greater amount of curve retention will allow increased predictability and greater control. The ability to retain a set curve is dependent on the material properties, namely the strain at yield. Deformation up to the yield point is elastically recoverable.

In some embodiments, the shaft of the distal end curvature comprises a shape memory material. In a specific example, the shape memory material is nickel titanium alloy Nitinol which exhibits a very high strain at yield. Fig. 21 illustrates the mechanical properties of Nitinol 52 compared to steel 51. The graph of Fig. 21 shows the stress (Y) vs. strain (X) curves of Nitinol 52 and steel 51. A material with a very high strain at yield is known as superelastic as it can resist elongation of up to 30% without permanent deformation. The distal end curvature comprising a superelastic material will exhibit very high curve retention. The hybrid dilator is able to be tracked through the vasculature without deforming the curve. In alternate embodiments, other superelastic or shape memory materials are used for example polymers such as polyether ether ketone (PEEK).

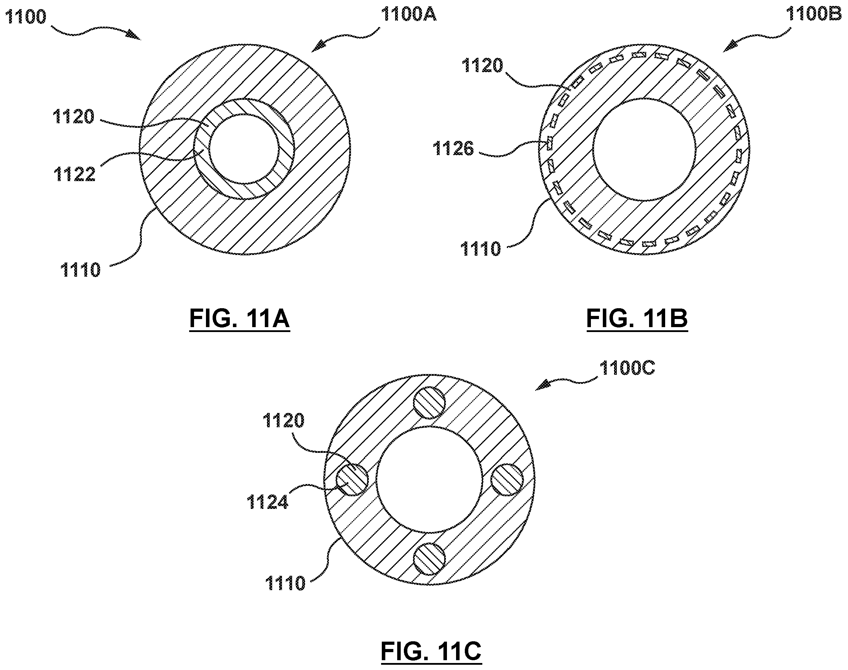

In the embodiments shown in Figures 11A - 11C, the hybrid dilator 1100 comprises a superelastic stiffening member 1120. The superelastic stiffening member 1120 is capable of elastically deforming while passing through vasculature and is capable of reverting to its original shape. In embodiments where the distal end comprises a superelastic stiffening member 1120, the hybrid dilator 1100 is capable of elastically deforming while passing through the vascular and capable of returning to its original curvature (i.e. it's curvature at time of manufacturing) at the target site more predictably.

Fig. 11A shows a cross section of the distal end curvature of a hybrid dilator 1110A. The hybrid dilator 1100A comprises a superelastic stiffening member 1120. In this embodiment, the superelastic stiffening member 1120 is a superelastic hypotube 1122. In a specific example, the hypotube 1122 is made of nitinol. The hypotube 1122 may be a continuous structure or have cut patterns.

Fig. 11B shows a cross section of the distal end curvature of a hybrid dilator 1110B. The hybrid dilator 1110B comprises a superelastic stiffening member 1120. In this embodiment, the superelastic stiffening member 1120 is braided wire 1126. In a specific example, the braided wire 1126 is made of nitinol.

Fig. 11C shows a cross section of the distal end curvature of a hybrid dilator 1110C. The hybrid dilator 1100C comprises a superelastic stiffening member 1120. In this embodiment, the superelastic stiffening member 1120 is a superelastic wire stiffener 1124. In a specific example, the wire stiffener 1124 is made of nitinol.

The hybrid dilator 1100 comprising a superelastic stiffening member 1120 will improve the curve retention of the device during the procedure. This will result in an improved ability to navigate anatomy without permanently deforming the device, and more predictable curve geometry when approaching the target site. This is beneficial during difficult access cases and where precise positioning is required.

In hybrid dilator 1100, a superelastic material such as Nitinol is used within the curved region. In one embodiment, this is constructed using standard catheter layup and thermal reflow techniques to embed the superelastic material into the shaft of the device. In alternate embodiments, the superelastic materials are incorporated through gluing, welding, or laminating within the shaft materials.

The superelastic material is positioned in the hybrid dilator 1100 where the curve is desired. After the superelastic material is positioned within this region, the curve geometry must be shape set. In one embodiment using the superelastic material Nitinol, the curve geometry is set by heating the Nitinol curve geometry a 400-550 degrees Celsius for 1-20 minutes. This is followed by quenching the part to room temperature. In some embodiments, there may be a sequence of heating and quenching steps to reach the final curve shape and shape memory needed.

### Methods of performing a transseptal procedure using a hybrid dilator and flexible puncture device

The hybrid dilator with a flexible puncturing device 800 such as an RF guidewire provides an improved workflow. This improves the efficacy of a procedure by eliminating steps from the workflow in procedures which may require specialty ancillary devices, such as specialty sheaths, to be used to deliver the end therapy devices once gaining access to the left atrium. Some examples of procedures requiring specialty ancillary devices are cryoablations, left atrial appendage occlusions (LAAO), transcatheter aortic valve replacement (TAVR), Mitral valve repairs, pulse field ablations, and RF ablations. These procedures commonly require the use of end-therapy devices which can only be delivered with sheaths having inner diameters greater than the sheaths used during transseptal puncture. This is because such end-therapy devices are larger in size than transseptal puncture devices, such as mechanical needles, RF needles, and RF guidewires. Specifically, transseptal puncture sheaths are 8Fr to 8.5Fr in diameter while some specialty sheaths, such as those used for cryoablation and LAAO, are sized 12Fr or larger. Due to the difference in the size of the sheaths for end-therapy devices and transseptal puncture devices, multiple exchanges are typically required in order to both perform the transseptal procedure (i.e., the procedure for puncturing the septum) and deliver the end-therapy device to the left atrium.

In some embodiments, the hybrid dilator is used in combination with a flexible puncturing device 800. Details of a flexible puncturing device and method of use are disclosed in International Publication No. WO2018/083599, entitled "METHODS AND DEVICES FOR PUNCTURING TISSUE", and US application US17/316,229 which are cited. The flexible puncturing device 800 (Fig. 11) may comprise an energy delivery device 812, such as an electrode 814, at the distal tip 810 that is operable to deliver energy, for example radiofrequency energy, in order to puncture the tissue. In some such embodiments, the distal tip 810 may be substantially atraumatic in order to reduce pressure exerted on the tissue and prevent inadvertent damage during the procedure. The atraumatic tip may be cylindrical, hemispherical, or a rounded dome. In some embodiments, the flexible puncturing device 800 may comprise an electrically insulative coating 840 with the energy delivery device 812 being exposed at the distal tip 810. In an alternative embodiment, the flexible puncturing device may comprise a relatively sharp distal tip in order to mechanically puncture the tissue (not shown).

In some embodiments, a flexible puncturing device 800 may be used to puncture tissue. The flexible puncturing device 800 may be in the form of a puncturing guidewire 802, for example a flexible 0.035" guidewire. In some embodiments, the flexible puncturing device may have a distal portion 823 wherein the stiffness is defined by a flexural rigidity of at least about 3.57 x10-6 Nm2 to about to about 5.95 x10-6 Nm2, for example about 4.76 x10-6 Nm2. The proximal portion 820 may have a flexural rigidity between 0.00107Nm2 to about 0.00179Nm2, for example 0.00143 Nm2.

The hybrid dilator 1000 is dimensioned to accommodate the RF guidewire. Specifically, the inner diameter of the hybrid dilator 1000 corresponds to the outer dimeter of the RF guidewire. In some embodiments, the inner diameter of the hybrid dilator 1000 may range from 0.0035" to 0.050", with a preferred inner diameter in the range of 0.038" to 0.44".

The stiffening member 1020 enables the hybrid dilator 1000 to be shaped either prior or during the procedure. The shapeability of hybrid dilator 1000 provides physicians with improved positioning on the septum while also providing increased reach of the distal tip.

When the hybrid dilator 1000 is used with a flexible puncturing device 800, the stiffening member 1020 provides stiffness to support the flexible puncturing device 800. Physicians may insert the hybrid dilator 1000 prior to manipulating the curved portion. During the procedure, physicians may then visualize the hybrid dilator 1000 and flexible puncturing device 800 using various imaging techniques to determine where the distal end of the hybrid dilator 1000 is positioned. If the distal end of the hybrid dilator 1000 is not positioned appropriately, the hybrid dilator 1000 may be withdrawn and shaped to a desired curvature before being reinserted. Alternatively, physicians may introduce the curvature prior to the procedure. Thus, the example of the workflow, described above, may include an additional step of shaping the hybrid dilator 1000 prior to the start of the procedure.

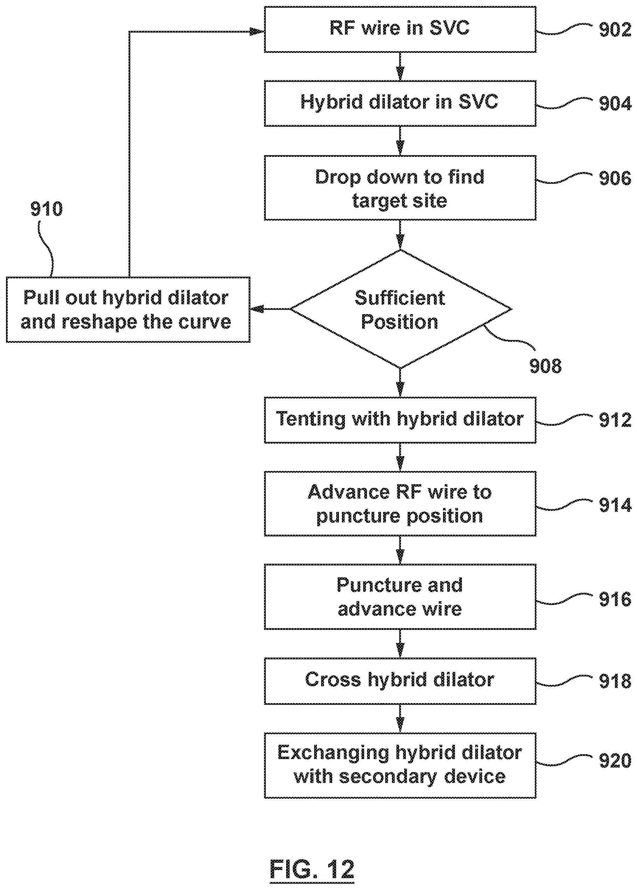

An example of the improved workflow with use of the present disclosure is illustrated in Fig. 12. This method comprises the steps of: (i) Advancing the flexible puncturing device such as an RF guidewire to the superior vena cava (SVC) 902. (ii) Advancing the hybrid dilator into the SVC overtop the RF guidewire 904. (iii) Dropping the hybrid dilator and RF guidewire onto the septum to a target site such as the fossa ovalis 906. (iv) Determining if the distal tip of the hybrid dilator is positioned on the target site 908. Where the distal tip of the hybrid dilator is positioned at the target site may be determined using various visualization methods such as fluoroscopy, electro-anatomical mapping, or echogenic markers. If the distal tip of the hybrid dilator is not in the correct position, (v) Removing the hybrid dilator and reshaping the curve of the hybrid dilator 910. The dilator is then reinserted and steps (ii) - (iv) are repeated until the distal tip of the hybrid dilator is positioned at the target site. Once the hybrid dilator is positioned correctly, (vi)Tenting the septum with distal tip of the hybrid dilator. 912. (vii) Advancing the RF guidewire such that the distal tip of the RF guidewire is contacting the FO 914. (viii) Puncturing the septum by energizing the RF guidewire and advancing the guidewire through the septum such that the distal tip is in the left atrium 916. Upon completing the puncture, the physician may confirm access into the left atrium through various methods such as fluoroscopy, electro-anatomical mapping, pressure differentials, contrast injection, or echogenic markers. (ix)Advancing the hybrid dilator across the septum 918 thereby enlarging the puncture. (x) Removing the hybrid dilator and advancing one or more secondary devices over the RF guidewire into the left atrium 920 to complete the desired procedure.

In procedures where the physician wishes to use a relatively large delivery sheath for complex procedures (for example for cryoablation or LAA occlusion) and knows they cannot cross with that product, the physician can now cross the septum using two devices, a flexible puncture device 800 and hybrid dilator such as the reshapeable hybrid dilator 1000. A number of exchanges are removed by reducing a sheath and dilator into one device, the hybrid dilator 1000, and reducing the guidewire and puncturing device into one device, the flexible puncture device 800. Thus, only two products are used to cross the septum and gain access to the left side of the heart. The flexible puncture device 800 can also allow the exchange with a secondary device wasting fewer products and streamlining the procedure.

### Steerable Hybrid Dilator

In some procedures, a fixed curve hybrid dilator may not provide the control or precision required to locate the target site. The reshapeable hybrid dilator 1000 has stiffening member 1020 which allows the physician to manipulate the curve to a desire angle, also known as reach. This control, however, can only be achieved while the device is outside of the body. In other words, once the device is inserted into the body, the curvature angle cannot be changed unless the device is removed from the body and manually reshaped by the physician. In cases with abnormal anatomy such as tortuous vasculature, large right atrium etc., the physician may remove the device multiple times to achieve the required curvature angle to perform the transseptal workflow. Each removal and insertion of a device into the body bears risk of introducing air embolisms into the vasculature.

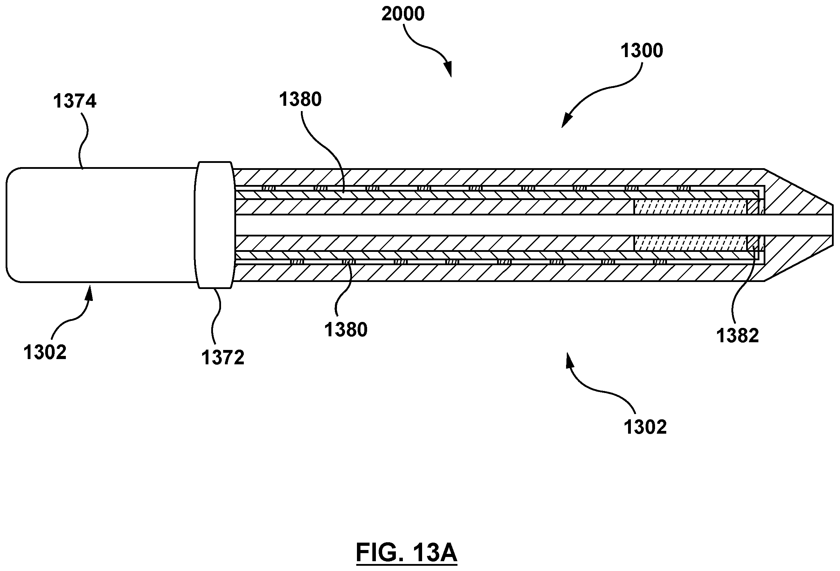

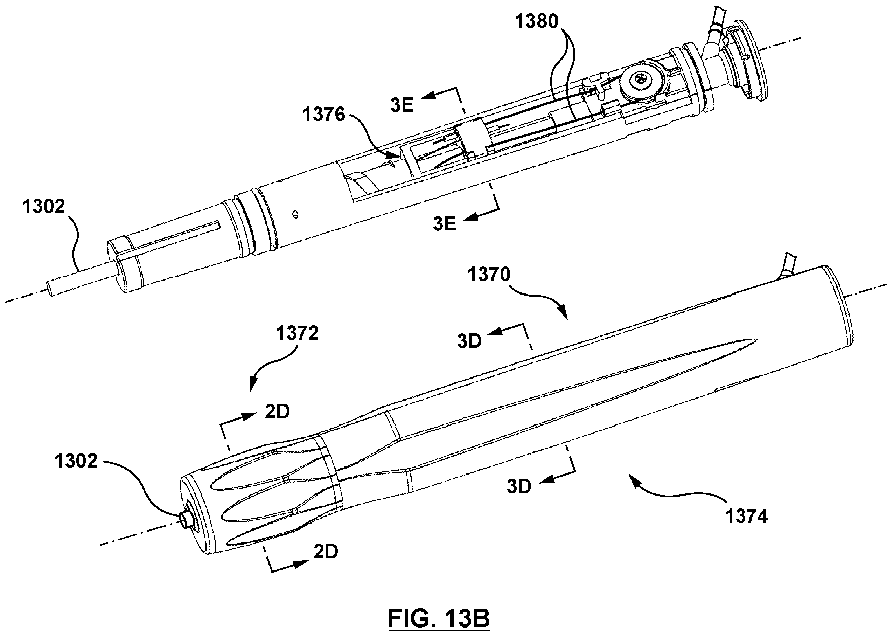

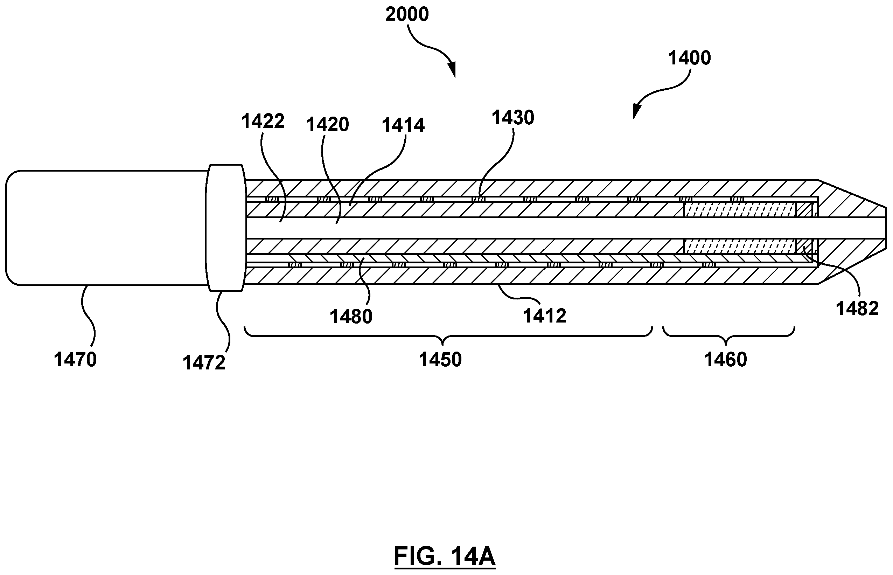

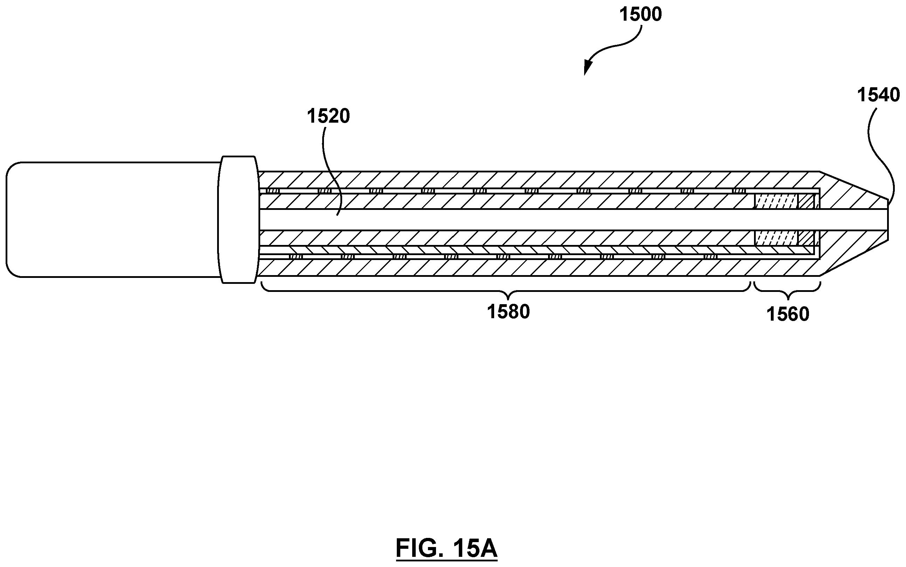

The distal curvature of a steerable hybrid dilator can be controlled by the user at the handle. In one embodiment of the present disclosure, a steerable control system or handle 1370 is provided for manipulating a hybrid dilator 1300. The steerable handle is disclosed in application PCT/IB2013/055013 which is cited. In a specific example, as shown in Figs. 13A and 13B, the handle 1370 is coupled to a shaft 1302 to enable a user to manipulate or steer the hybrid dilator 1300 in a desired direction during use. The handle 1370 comprises a handle control 1372 that is rotatably coupled to a handle housing 1374. The handle control 1372 is rotatable about the longitudinal axis of the handle 1370 and rotates with respect to housing 1374. In operation, the rotation of the handle control 1374 in a first rotational direction allows the user to steer or deflect the shaft 1302 in a first direction, whereas the rotation of the handle control 1374 in a second rotational direction allows the user to steer or deflect the shaft 1302 in a second direction. In alternate embodiments, the hybrid dilator has unidirectional control (Figs. 14A and 15A). In some embodiments as described herein, the bi-directional steerable catheter described is operable to be deflected in two different deflection directions, a first and a second deflection direction. In other embodiments, the bi-directional steerable catheter is configured to (or has the internal workings that enable it to) deflect in two different deflection directions; however, the deflection of the catheter in one of its deflection directions is limited or restricted such that the observed deflection of the catheter is limited to a single deflection direction (relative to the starting, or neutral, position). Thus, in some embodiments a unidirectional control system is provided for a bi-directional steerable catheter to provide a unidirectional steerable catheter including at least two pull wires.

The rotation of the handle control 1372 is converted into a deflection of the shaft 1302 via a slide assembly 1376, shown in Fig. 13B. Generally, handle control 1372 is co-operatively engaged with the slide assembly 1376 which is housed within a lumen defined by the handle housing 1374. In a specific example, the handle control 1372 is threadably engaged with slide assembly 1376. The rotation of handle control 1472 causes a corresponding linear translation of the slide assembly 1376 within the housing 1370. This translation of the slide assembly 1376 is converted into a tensioning of the pull wires 1380 coupled to the slide assembly 1376 and thereby resulting in a deflection of the shaft 1302.

The steerable hybrid dilator 1300 has increased precision and improved ability to locate the target tissue.

### Steerable and Reshapeable Hybrid Dilator

Current steerable catheters only offer the control of distal tip of the catheter also known as precision. In cases of abnormal anatomy, the deflection afforded by the steerable catheter may be insufficient to provide the necessary reach. Conversely, reshapeable, fixed curved catheters only offer control of the broad curve also known as reach. In cases with abnormal anatomy, the physician may remove the device multiple times to achieve the required curvature angle to perform the transseptal workflow.

The inventors of the present invention have identified the limitations of each device and discovered systems to overcome these limitations.

Fig. 14A shows a catheter 2000 that has a reshapeable proximal portion 1450, and a deflectable distal portion 1460. In the specific embodiment of Fig. 14A, the catheter is a hybrid dilator 1400 in accordance with the invention. Alternate embodiments of the catheter 2000 are a sheath, dilator, needle, etc. (not shown). The physician is able to manipulate the reshapeable proximal portion 1450 when the device is outside the body and is able to manipulate the curve of the deflectable distal portion 1460 while the catheter 2000 is within the body.

The combination of a proximal portion configured to be reshapeable, and a distal portion configured to be deflectable allows the physician to reshape the proximal portion to achieve a desired reach and steer the distal portion for precision while inside the anatomy.

The catheter 200 of Fig. 14A is a reshapeable and steerable hybrid dilator 1400. The reshapeable proximal portion 1450 of the hybrid dilator 1400 comprises a stiffening member 1420. The stiffening member 1420 enables the proximal portion of hybrid dilator to be shaped either prior or during the procedure. The stiffening member 1420 plastically deforms while being manipulated by the physician. Once the physician has curved the proximal portion 1450 of hybrid dilator 1400, the stiffening member 1420 has sufficient rigidity to resist relaxation of the curve. The reshapeable proximal portion 1450 extends between the handle 1470 and the deflectable distal portion 1460. In the embodiment of Fig. 14A, the reshapeable proximal portion 1450 extends from the handle 1470 and ends proximal to the deflectable distal portion 1460.

In some embodiments, the stiffening member 1420 is hypotube 1422 made of metal such as stainless steel. In alternate embodiments, the stiffening member 1420 is heterogenous formed of multiple layers and segments of metal and/or plastic (not shown). An additional layer of plastic may be included for increased rigidity. The inner layer 1414 may consist of one or multiple segments of plastic with different properties. In the proximal portion 1450, the inner layer may consist of a harder plastic for example a plastic having a Shore D>60 such as HDPE and Pebax 60D-80D.

The deflectable distal portion 1460 of the hybrid dilator 1400 can be controlled by the user at the handle 1470. The deflectable distal portion 1460 is compliant and flexible allowing the distal portion 1460 to curve. The hybrid dilator 1400 has a steering mechanism comprising a steering handle 1470 which is operatively connected to at least one control wire 1480 for steering the deflectable distal end portion 1460.

The deflectable distal portion 1460 comprises an attachment point for a pull wire 1480. In a specific embodiment, the pull wire 1480 is attached to the distal portion 1460 via a pull ring 1482. The pull wire 1480 extends substantially along the length of the hybrid dilator 1400. The distal end of the pull wire is attached to the pull ring 1482. The proximal end of the pull wire 1480 is fixed relative to a translating component housed within the steering handle 1470. A control device such as handle control 1472 controls the translating component and thus controls the pull wire 1480. In a specific embodiment, the rotation of the handle control 1472 translates into linear movement of the translating component. The linear movement along the handle's longitudinal axis applies tension at the proximal end of the pull wire 1480, thereby causing the distal portion of the shaft to deflect. In a specific embodiment, the handle control 1472 automatically locks the curvature angle of the distal portion 1460 when released by the user. In other words, the physician does not require to apply constant force to maintain a curvature in the distal portion 1460.

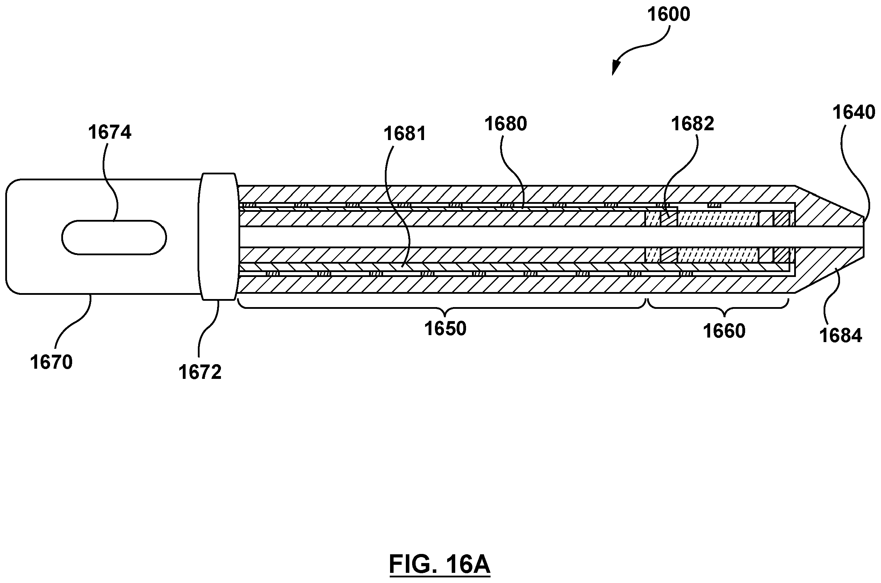

The pull wire 1480 of the deflection mechanism is installed into the handle. This may consist of the sole or combined construction of pulleys, rack and pinions, rotating gears, motors, etc. In a specific embodiment, a single pull wire 1480 and pull ring 1482 provides deflection in one plane. In other words, the deflection is unidirectional. The proximal end of the pull wire 1480 may be mechanically bonded to a travelling member within the handle such as a block. A rotating or linear control may be used to move the travelling member axially along the length of the handle where this motion applies a tensile force on the pull wire 1480 which causes the distal portion 1460 to deflect. Handle control 1472 is an example of a rotating control. The second handle control 1674 (Fig. 16A) is an example of a linear control.

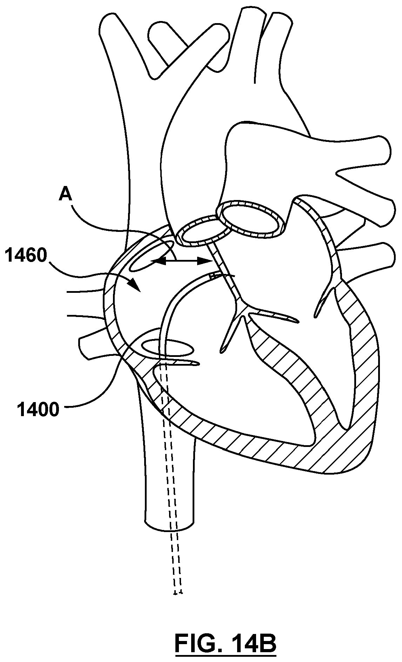

In the embodiment of Fig. 14A, the distal portion 1460 is between approximately 5-10 cm. In other words, the steerable curve is achieved across this portion. The physician may manipulate the proximal portion 1450 while the catheter is out of the body to achieve the desired reach of the hybrid dilator 1400. In this embodiment, the hybrid dilator 1400 can be deflected within the body to further improve the reach of the hybrid dilator and provide precision to the distal end of the hybrid dilator 1400. The reach of the hybrid dilator 1400 of Fig. 14A is shown in Fig. 14B (arrow A).

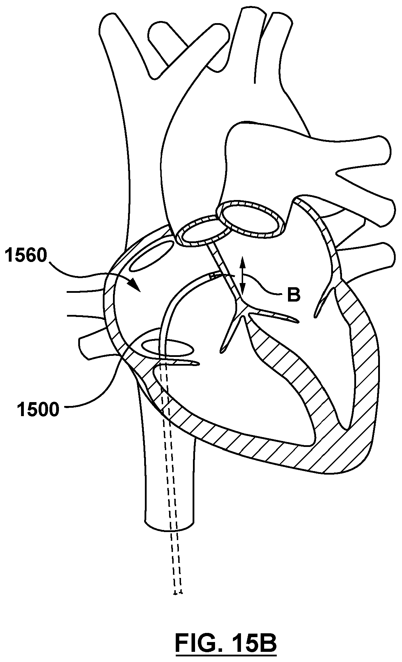

In the embodiment of 15A, the distal portion 1560 of the hybrid dilator 1500 is between approximately 2-4 cm. In this embodiment, the steerable curve is achieved across a smaller length than the embodiment of Fig. 14A. In other words, the stiffening member 1520 extends further distally on hybrid dilator 1500 creating a longer shapeable proximal segment 1580. Therefore, the deflection point (i.e. the point where the curvature occurs) is further distal on the hybrid dilator 1500 thereby the deflection imparted via the deflection mechanism in the handle 1470 will change the distal most tip. A smaller distal portion 1560 offers increased precision of the distal end. The physician may manipulate the proximal portion 1550 while the catheter is out of the body to achieve the desired reach of the hybrid dilator 1500 then control the precision of the distal end while the hybrid dilator 1500 is inside the body. The precision control of the hybrid dilator 1500 can be seen in Fig. 15B (arrow B).

The hybrid dilator 1400 of Fig. 14A additionally comprises a torque layer 1430. The torque layer 1430 improves the torqueability of the device. The torque layer is typically a braided material. The braided material may have a pitch or picks per inch (PPI) in a range from 20-70 PPI (1 inch = 25,4 mm). The PPI influences the torquability of the shaft. The PPI may be constant along the shaft or may vary. The torque layer 1430 is positioned between the inner layer 1412 and the and outer layer 1014. The pull wire 1480 is encompassed within the layer of braiding.

In the specific embodiments of Fig. 14A, the inner layer 1414 of the hybrid dilator 1400 is comprised of different materials. In a specific embodiment, the inner layer 1414 of the proximal portion 1450 is a harder plastic than the inner layer 1414 of the distal portion 1460. The proximal portion 1450 inner layer may be made of HDPE, higher durometer Pebax (>60D) and/or Nylon. The distal portion 1460, inner layer 1414 may be a softer plastic for example a plastic with a Shore D<50 such as LDPE or a lower durometer Pebax (<50D). These segments of plastic may be thermally bonded together. In alternate embodiments, Blends of HDPE and LDPE of varying ratios (e.g. 80/20, 50/50) may also be used as a segment along the lengths of the shaft. In alternate embodiments, the inner layer 1414 is comprised of a single material (not shown).

In some embodiments, the outer layer 1412 has a lower durometer than the stiffening member 1420. In other words, the outer layer 1412 is softer than the stiffening member 1420. In such embodiments, the stiffening member 1420 provides the required shapeability while the outer layer provides a softer surface that would not cause damage to vessels. The softer outer layer is smoother and therefore easier to navigate vasculature. In some embodiments the outer layer 1412 is formed of a single material. In other embodiments, the outer layer 1412 is formed of multiple layers of materials. In some embodiments, the outer layer 1412 is a material with a lower durometer than the inner layer 1414. For example, the outer layer 1412 has a hardness of Shore D <50. The ratio of the thickness ratio of the outer layer 1412 and the inner layer 1414 dictates the stiffness and torque transmission of the hybrid dilator shaft. In some embodiments, the outer layer is LDPE, an HDPE/LDPE blend or a Pebax D50 or less. A bonding step may be performed to bond the outermost shaft layer to the remainder of the assembly.

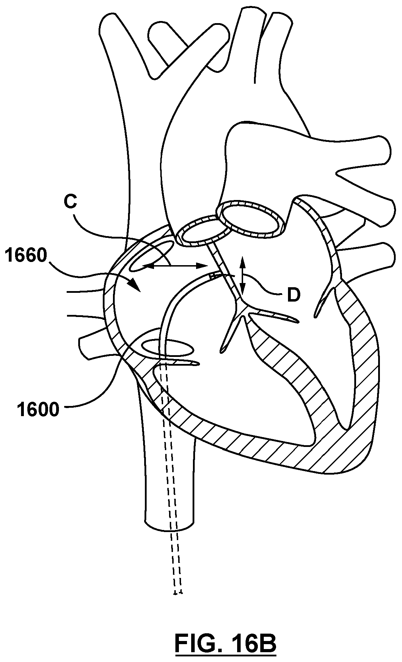

The catheter 2000 in Fig 16A is hybrid dilator 1600 comprising two deflection points. In this specific embodiment, hybrid dilator 1600 comprises two pull rings 1682, 1684. The first pull ring 1682 is located approximately 5-10 cm from the distal tip 1640. The second pull ring 1684 is located approximately 2-4 cm from the distal tip 1640. The first pull ring 1682 is operatively connected to a first control such as handle control 1674 via pull wire 1680. The second pull ring 1684 is operatively connected to a second control such as handle control 1672 via pull wire 1682. The handle controls 1672 and 1674 are controlled independently from one another. In this specific embodiment, the handle control 1674 is a linear control which controls the proximal pull ring 1682. This control improves the reach of the hybrid dilator 1600. The reach (arrow C) can be seen in Fig. 16B. The handle control 1672 is a rotational control which controls the distal pull ring 1684. This control improves the control of the distal tip 1640 or in other words, the precision of the hybrid dilator 1600. The precision (arrow D) can be seen in Fig. 16B. In other embodiments, the handle control 1674 controls the distal pull ring 1684 and the handle control 1672 controls the proximal pull ring 1682 (not shown).

### Methods of performing a transseptal procedure using a steerable hybrid dilator and flexible puncture device

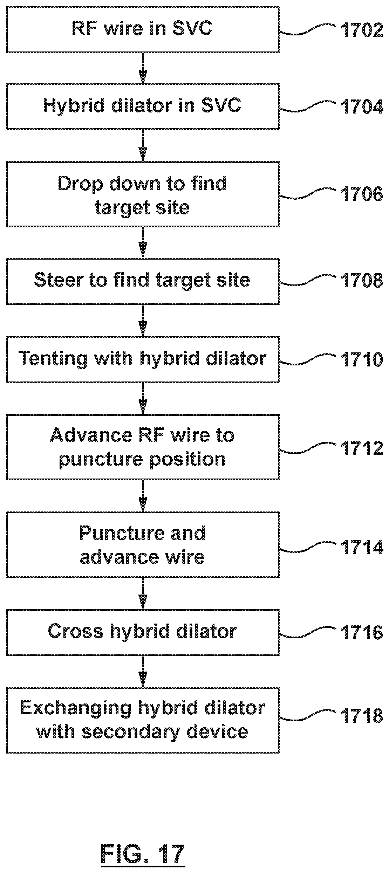

An example of the workflow of the steerable hybrid dilator with a flexible puncture device is illustrated in Fig. 17. This method comprises the steps of: (i) Advancing the flexible puncture device such as an RF guidewire to the SVC 1702. Alternatively, the flexible puncturing device may be a sharp-tipped guidewire. (ii) Advancing the hybrid dilator into the SVC overtop the RF guidewire 1704. (iii) Dropping the hybrid dilator and RF guidewire onto the septum to a target site such as the fossa ovalis 1706. (iv)Steering the distal tip of the hybrid dilator to the target site 1708. This step may be assisted using various visualization methods such as fluoroscopy, electro-anatomical mapping, or echogenic markers. Once the hybrid dilator is positioned correctly, (v)Tenting the septum with distal tip of the hybrid dilator 1710. (vi) Advancing the RF guidewire such that the distal tip of the RF guidewire is contacting the target site 1712. (vii) Puncturing the septum by energizing the RF guidewire and advancing the guidewire through the septum such that the distal tip is in the left atrium 1714. Upon completing the puncture, the physician may confirm access into the left atrium through various methods such as fluoroscopy, electro-anatomical mapping, pressure differentials, contrast injection, or echogenic markers. (viii)Advancing the hybrid dilator across the septum 1716 enlarging the puncture. (ix) Removing the hybrid dilator and advancing one or more secondary devices over the RF guidewire into the left atrium 1718 to complete the desired procedure.

In embodiments where the steerable hybrid dilator comprises a stiffening member such as hybrid dilator 1400, the hybrid dilator 1400 may be shaped either prior or during the procedure. This additional step allows the physician to match the reach of the hybrid dilator 1400 to the specific anatomy. In an embodiment of the method, prior to tenting the septum, the physician may remove the hybrid dilator and reshape the hybrid dilator to better access the target tissue. After reshaping, the physician would insert the hybrid dilator and continue from step 1704.

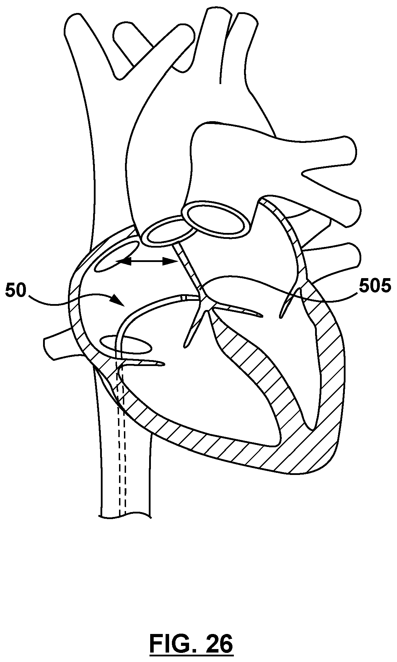

In some instances of a conventional transeptal puncture (Fig. 6A and 6B), the fixed curve transseptal system (needle, dilator and sheath) may not align with the fossa ovalis on the septum. For example, during the drop-down procedure, the dilator and sheath assembly 50 has dropped too low (i.e. inferiorly) on the septum 505 as seen in Fig. 26. In such an event, a safe and effective transseptal puncture cannot be performed. To return to the fossa, the drop-down procedure would have to be performed again. This often involves removing the dilator and sheath assembly 50, advancing the guidewire into the SVC, advancing the sheath 20 and dilator 40 over the guidewire into the SVC, exchanging the guidewire with a needle, and performing the drop-down procedure to attempt to land on the fossa ovalis.

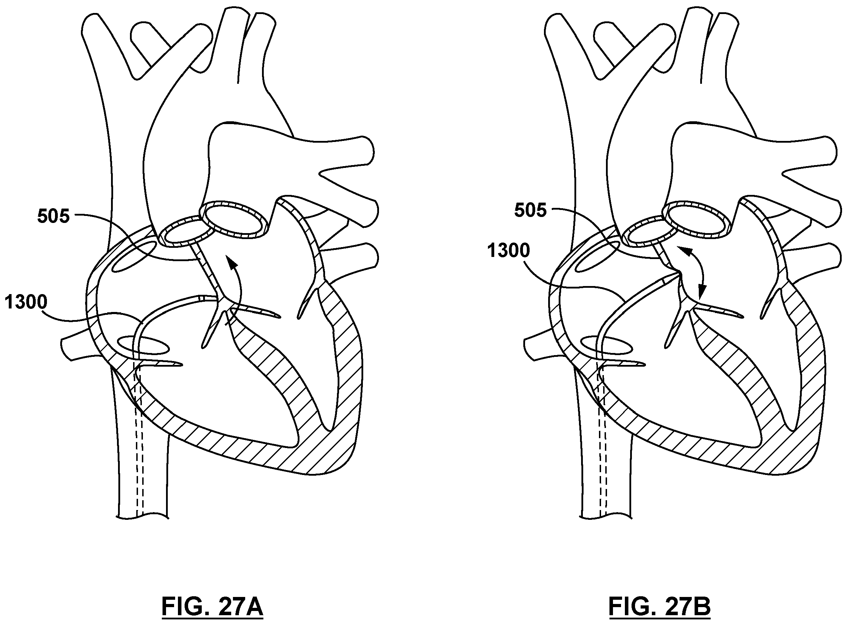

A steerable hybrid dilator 1300 and flexible puncture device increases the precision of the transseptal puncture and can eliminate the exchanges required in circumstances where the dilator is not positioned on the desire location of the septum 505. In the scenario where the steerable hybrid dilator falls too low (i.e. inferiorly) on the septum 505 during the drop-down step (Fig. 27A), the steerable hybrid dilator 1300 may be maneuvered and steered to change the distal curvature such that it moves superiorly onto the fossa ovalis of the septum 505. This is advantageous over the conventional transseptal puncture procedure as the steerable hybrid dilator 1300 does not have to be withdrawn from the septum 505 or the body to correct for a low position on the septum 505. In some procedures, the target puncture site is a superior position on the fossa ovalis. Fig. 27B shows an embodiment where the steerable hybrid dilator 1300 is on the fossa but is at a lower (inferior) position than desired. The physician is able to steer the steerable hybrid dilator 1300 to a superior position on the fossa without removing the steerable hybrid dilator 1300 from the fossa ovalis. This increases the precision of the transseptal puncture which may improve the efficacy of the therapy.

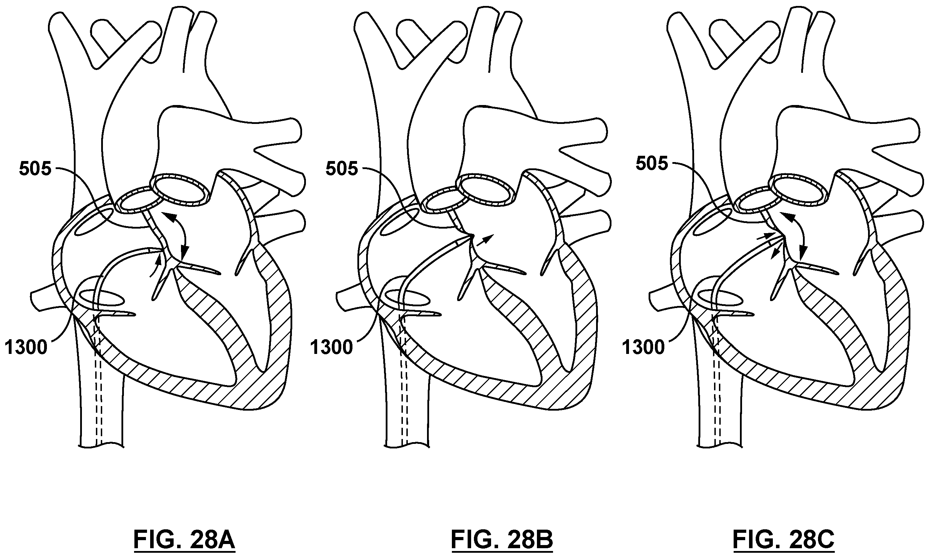

When positioned on the fossa ovalis, the steerable hybrid dilator 1300 may be steered such that it changed the amount of the fossa that is tented (Fig. 28A and Fig. 28B). This in-situ control may improve the safety and efficacy of the transseptal puncture as it can adjust the tent to accommodate for variabilities in the anatomy. For example, if the physician feels they have excessive or insufficient tenting to perform an effective transseptal puncture, they may change the distal curve (Fig. 28C) to decrease or increase the tenting. In the embodiment of Fig. 28C, the right atrium 501 may be larger and the distance between the septum 505 and right atrium wall 503 is greater than the normal anatomy. As such, even if the steerable hybrid dilator 1300 is on the fossa ovalis, the physician may need to increase the force applied to the fossa to achieve a desirable tent for puncture. This may be achieved by steering the steerable hybrid dilator 1300.

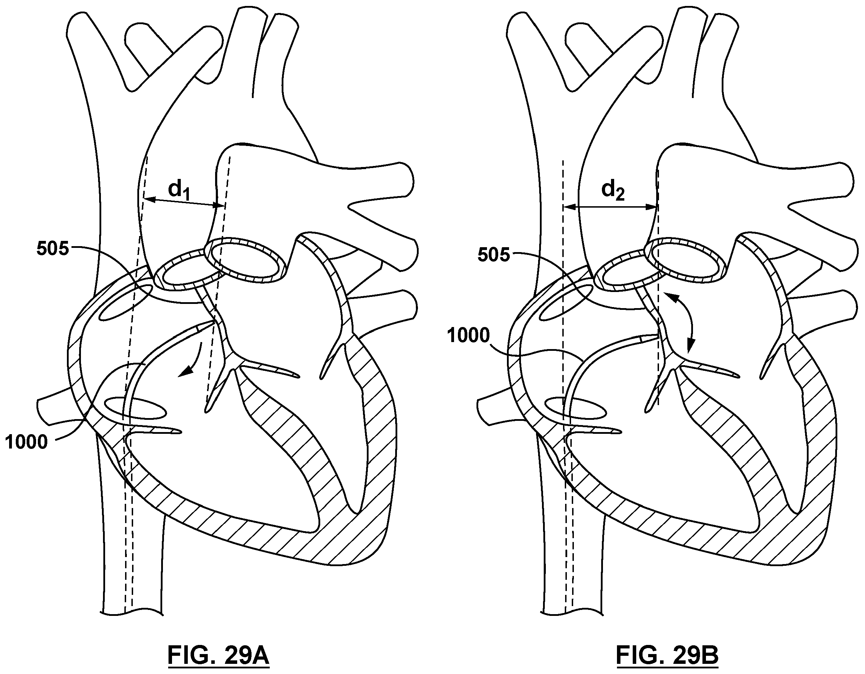

Fig. 29A shows an alternate embodiment where the hybrid dilator 1000 does not have sufficient reach to contact the septum 505 (i.e. the curve of the dilator 1300 does not provide sufficient lateral distance between the distal tip of the dilator and the axis of the straight portion). In a reshapeable hybrid dilator 1000, the dilator may be withdrawn from the body to be manually re-shaped (i.e. physical manipulation of the distal curvature by hand to increase the reach) then reintroduced into the SVC to perform the drop-down. In a steerable hybrid dilator 1300, the distal curve may be controlled through the steering mechanism to increase the reach. In a reshapeable steerable hybrid dilator 1400, a combination of manually re-shaping the dilator outside the body and steering the dilator in-situ can maximize the reach and control of the dilator. By re-shaping and/or steering, the hybrid dilator's 1000 reach can be extended and the accuracy of puncturing location can be improved. Fig. 29A show the hybrid dilator 1000 with a reach of d₁ unable to reach the septum 505. After re-shaping, steering or a combination of the two, the hybrid dilator is able to reach the septum 505 with a reach of d₂ as shown in Fig. 29B.

Additionally, during some procedures, the physician may desire a specific puncture site based on the end therapy procedure. For a mitral valve repair, the physician may want to puncture the septum at a superior position on the fossa. Meanwhile, for a pulmonary vein ablation, the physician may want to puncture the septum at an anterior position on the fossa. By re-shaping and/or steering the hybrid dilator 100, the physician is able to direct and/or steer the distal tip of the hybrid dilator 100 to the desired position on the septum 505. Furthermore, after the puncture has been performed, the physician may direct the hybrid dilator 100 towards the particular anatomical feature. A steerable hybrid dilator provides additional control for the physician to accurately position the device after the puncture.

### RO marker

In some embodiments, the hybrid dilator comprises a marker at the tapered distal end. The marker may be fluoroscopic and/or echogenic. The marker indicates the distal tip location as well as the apex of the taper. In some embodiments, the marker is a radiopaque marker band. In other embodiments, the marker is a changing material with varying radiopaque properties at distal tip as well as the apex of the taper.

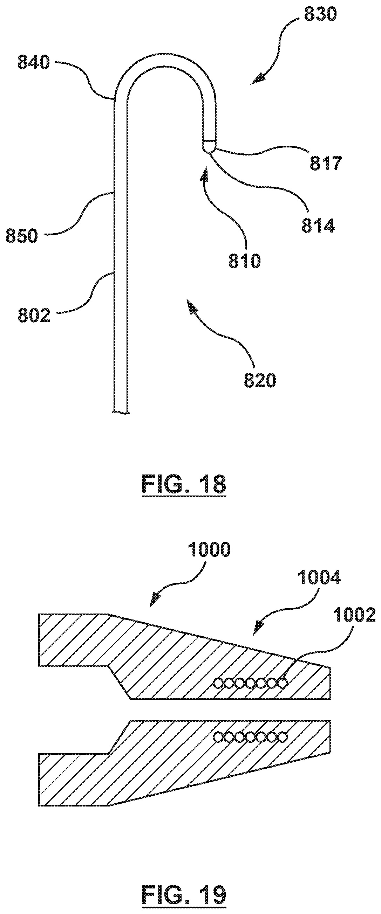

With reference now to Figure 19, the reinforced dilator 1000 may include a radiopaque marker 1002 located at the distal tip 1004. This radiopaque marker 1002 may be in the form of a radiopaque band or coil embedded within one of the polymer layers. The radiopaque marker 1002 enables physicians to visualize the distal tip 1004 of the enhanced dilator 1000 throughout the procedure. (e.g. platinum, gold, tungsten, and/or barium sulfate-filled polymer). In other embodiments an alternate radiopacifier is embedded in the polymer material such as BiOCL. In some embodiments, the BiOCL is <25%. In another embodiment, a change in radiopaque materials is used to visualize the distal tip 1004. Fig. 20 illustrates an embodiment of the distal tip of a hybrid dilator 1000 with a radiopaque marker 1002 under fluoroscopy. Fig. 20 additionally shows an embodiment of the distal tip of a hybrid dilator 1000 with a radiopacifier material (such as BiOCL) embedded in the polymer material 1003.

As such, in accordance with embodiments of the present disclosure, a method is provided for streamlining the procedural workflow by providing a hybrid dilator that combines the functionalities of a conventional transseptal sheath and dilator assembly. With the hybrid dilator of the present invention a reduced number of devices may be required in order to complete the transseptal procedure, which enhances procedural efficiency while reducing procedural time and complexity.

The embodiment(s) of the invention described above are intended to be exemplary only. The scope of the invention is therefore intended to be limited solely by the scope of the appended claims.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims. In addition, citation or identification of any reference in this application shall not be construed as an admission that such reference is available as prior art to the present invention.

## Claims

1. A hybrid dilator (1400) for use with a crossing device in tissue puncturing procedures, the hybrid dilator (1400) comprising:
a dilator body comprising
a dilator shaft defining a lumen for receiving a crossing device therethrough, the dilator shaft being structured to provide support for the crossing device when the crossing device is used to create a puncture in a tissue, the dilator shaft comprising a proximal portion (1450) and a distal portion (1460);
the proximal portion (1450) comprising at least one stiffening member (1420), wherein the at least one stiffening members (1420) is reshapeable;
the distal portion (1460) comprising a distal tip having an outer diameter which tapers down to an outer diameter of the crossing device for providing a smooth transition between the crossing device and the distal tip when the crossing device is inserted through the lumen and protrudes beyond the distal tip;
a deflectable distal end portion; and
at least one pull wire (1480); and
a steering handle connected to a proximal end portion of the dilator body, the steering handle operatively connected to the at least one pull wire (1480) for steering the deflectable distal portion of the dilator body in at least one direction,
wherein the stiffening member (1420) is plastically deformable by manual manipulation to have a curved shape, wherein the stiffening member (1420) has sufficient rigidity to resist relaxation of the curved shape and to hold the curved shape when navigated through vasculature to a target site.

2. The hybrid dilator (1400) of claim 1 wherein the stiffening member (1420) is a hypotube.

3. The hybrid dilator (1400) of claim 1 where the stiffening members (1420) is at least one stiffening wire.

4. The hybrid dilator (1400) of claim 1 wherein the hybrid dilator (1400) comprises two stiffening members (1420).

5. The hybrid dilator (1400) of claim 4, wherein a first stiffening member (1420) is a hypotube and a second stiffening member (1420) is at least one stiffening wire.

6. The hybrid dilator (1400) of any one of claims 1 to 5, wherein the hybrid dilator (1400) further comprises a torque layer (1430).

7. The hybrid dilator (1400) of claim 6 wherein the torque layer (1430) is a braided material.

8. The hybrid dilator (1400) of any one of claims 1 to 7 wherein the deflectable distal end portion comprises at least one pull ring (1482) attached to the at least one pull wire (1480).

9. The hybrid dilator (1400) of claim 8 wherein the hybrid dilator (1400) comprises two pull wires (1480)and the deflectable distal end portion comprises two pull rings (1482), a first pull ring (1482) attached to a first pull wire (1480) and a second pull ring (1482) distal to the first pull ring (1482) attached to a second pull wire (1480).

10. The hybrid dilator (1400) of claim 9 wherein the handle comprises a first handle control (1472) operatively connected to the first pull wire (1480) for steering the deflectable distal potion and a second handle control (1472) operatively connected to the second pull wire (1480) for steering the deflectable distal portion (1460).

11. The hybrid dilator (1400) of any one of claims 1 to 10 wherein the stiffening member (1420) terminates at a distal end of the proximal portion (1450).

12. The hybrid dilator (1400) of any one of claims 1 to 11 wherein the dilator shaft has an outer diameter from about 12 French to about 20 French.

13. The hybrid dilator (1400) of any one of claims 1 to 12 wherein the dilator shaft comprises an outer layer (1414) and an inner layer (1412).

14. The dilator of claim 13 wherein the outer layer (1414) is fixed to the inner layer (1412), and wherein the stiffening member (1420) is positioned between the inner layer (1412) and the outer layer (1414).

15. The dilator of claim 14 wherein the outer layer (1414) is fixed to the inner layer (1412) by a reflow process.

## Patentansprüche

1. Hybriddilatator (1400) zur Verwendung mit einer Kreuzungsvorrichtung bei Gewebepunktionsverfahren, wobei der Hybriddilatator (1400) umfasst:
einen Dilatatorkörper, umfassend
einen Dilatatorschaft, der ein Lumen zur Aufnahme einer Kreuzungsvorrichtung dadurch definiert, wobei der Dilatatorschaft so konstruiert ist, dass er der Kreuzungsvorrichtung Halt bietet, wenn die Kreuzungsvorrichtung verwendet wird, um eine Punktion in einem Gewebe herzustellen, und wobei der Dilatatorschaft einen proximalen Abschnitt (1450) und einen distalen Abschnitt (1460) umfasst;
wobei der proximale Abschnitt (1450) mindestens ein Versteifungselement (1420) umfasst, wobei das mindestens eine Versteifungselement (1420) verformbar ist;
wobei der distale Abschnitt (1460) eine distale Spitze umfasst, die einen Außendurchmesser aufweist, der sich auf einen Außendurchmesser der Kreuzungsvorrichtung verjüngt, um einen sanften Übergang zwischen der Kreuzungsvorrichtung und der distalen Spitze bereitzustellen, wenn die Kreuzungsvorrichtung durch das Lumen eingeführt wird und über die distale Spitze hinausragt;
einen auslenkbaren distalen Endabschnitt; und
mindestens einen Zugdraht (1480); und
einen Lenkgriff, der mit einem proximalen Endabschnitt des Dilatatorkörpers verbunden ist, wobei der Lenkgriff funktionsmäßig mit dem mindestens einen Zugdraht (1480) verbunden ist, um den auslenkbaren distalen Abschnitt des Dilatatorkörpers in mindestens eine Richtung zu lenken,
wobei das Versteifungselement (1420) durch manuelle Manipulation plastisch verformbar ist, um eine gekrümmte Form anzunehmen, wobei das Versteifungselement (1420) eine ausreichende Steifigkeit aufweist, um einer Relaxation der gekrümmten Form entgegenzuwirken und die gekrümmte Form beizubehalten, wenn es durch das Gefäßsystem zu einer Zielstelle geführt wird.

2. Hybriddilatator (1400) nach Anspruch 1, wobei das Versteifungselement (1420) ein Hypotube ist.

3. Hybriddilatator (1400) nach Anspruch 1, wobei das Versteifungselement (1420) mindestens ein Versteifungsdraht ist.

4. Hybriddilatator (1400) nach Anspruch 1, wobei der Hybriddilatator (1400) zwei Versteifungselemente (1420) umfasst.

5. Hybriddilatator (1400) nach Anspruch 4, wobei ein erstes Versteifungselement (1420) ein Hypotube ist und ein zweites Versteifungselement (1420) mindestens ein Versteifungsdraht ist.

6. Hybriddilatator (1400) nach einem der Ansprüche 1 bis 5, wobei der Hybriddilatator (1400) ferner eine Drehmomentschicht (1430) umfasst.

7. Hybriddilatator (1400) nach Anspruch 6, wobei die Drehmomentschicht (1430) ein geflochtenes Material ist.

8. Hybriddilatator (1400) nach einem der Ansprüche 1 bis 7, wobei der auslenkbare distale Endabschnitt mindestens einen Zugring (1482) umfasst, der an dem mindestens einen Zugdraht (1480) befestigt ist.

9. Hybriddilatator (1400) nach Anspruch 8, wobei der Hybriddilatator (1400) zwei Zugdrähte (1480) umfasst und der auslenkbare distale Endabschnitt zwei Zugringe (1482) umfasst, wobei ein erster Zugring (1482) an einem ersten Zugdraht (1480) befestigt ist und ein zweiter Zugring (1482), der distal zum ersten Zugring (1482) angeordnet ist, an einem zweiten Zugdraht (1480) befestigt ist.

10. Hybriddilatator (1400) nach Anspruch 9, wobei der Griff eine erste Griffsteuerung (1472), die funktionsmäßig mit dem ersten Zugdraht (1480) verbunden ist, um den auslenkbaren distalen Abschnitt zu steuern, und eine zweite Griffsteuerung (1472) umfasst, die funktionsmäßig mit dem zweiten Zugdraht (1480) verbunden ist, um den auslenkbaren distalen Abschnitt (1460) zu steuern.

11. Hybriddilatator (1400) nach einem der Ansprüche 1 bis 10, wobei das Versteifungselement (1420) an einem distalen Ende des proximalen Abschnitts (1450) endet.

12. Hybriddilatator (1400) nach einem der Ansprüche 1 bis 11, wobei der Dilatatorschaft einen Außendurchmesser von etwa 12 French bis etwa 20 French aufweist.

13. Hybriddilatator (1400) nach einem der Ansprüche 1 bis 12, wobei der Dilatatorschaft eine äußere Schicht (1414) und eine innere Schicht (1412) umfasst.

14. Dilatator nach Anspruch 13, wobei die äußere Schicht (1414) an der inneren Schicht (1412) fixiert ist und wobei das Versteifungselement (1420) zwischen der inneren Schicht (1412) und der äußeren Schicht (1414) angeordnet ist.

15. Dilatator nach Anspruch 14, wobei die äußere Schicht (1414) durch ein Reflow-Verfahren an der inneren Schicht (1412) fixiert ist.

## Revendications

1. Dilatateur hybride (1400) pour une utilisation avec un dispositif de traversée lors de procédures de ponction de tissu, le dilatateur hybride (1400) comprenant :
un corps de dilatateur comprenant :
un arbre de dilatateur qui définit une lumière pour recevoir au travers un dispositif de traversée, l'arbre de dilatateur étant structuré de manière à constituer un support pour le dispositif de traversée lorsque le dispositif de traversée est utilisé pour créer une ponction dans un tissu, l'arbre de dilatateur comprenant une partie proximale (1450) et une partie distale (1460) ;
la partie proximale (1450) comprenant au moins un élément de raidissement (1420), dans lequel l'au moins un élément de raidissement (1420) peut être reconfiguré en termes de forme ;
la partie distale (1460) comprenant une pointe distale qui présente un diamètre externe qui est réduit progressivement jusqu'à un diamètre externe du dispositif de traversée pour assurer une transition lisse entre le dispositif de traversée et la pointe distale lorsque le dispositif de traversée est inséré au travers de la lumière et fait saillie au-delà de la pointe distale ;
une partie d'extrémité distale pouvant être orientée ; et
au moins un fil de traction (1480) ; et
une poignée de commande de direction qui est connectée à une partie d'extrémité proximale du corps de dilatateur, la poignée de commande de direction étant connectée de manière opérationnelle à l'au moins un fil de traction (1480) pour commander la direction de la partie d'extrémité distale pouvant être orientée du corps de dilatateur dans au moins une direction,
dans lequel l'élément de raidissement (1420) peut être déformé plastiquement par manipulation manuelle de telle sorte qu'il présente une forme incurvée, et dans lequel l'élément de raidissement (1420) présente une rigidité suffisante pour résister à la relaxation de la forme incurvée et pour conserver la forme incurvée lorsqu'il est guidé au travers du système vasculaire jusqu'à un site cible.

2. Dilatateur hybride (1400) selon la revendication 1, dans lequel l'élément de raidissement (1420) est un tube hypodermique.

3. Dilatateur hybride (1400) selon la revendication 1, dans lequel l'élément de raidissement (1420) est au moins un fil de raidissement.

4. Dilatateur hybride (1400) selon la revendication 1, dans lequel le dilatateur hybride (1400) comprend deux éléments de raidissement (1420).

5. Dilatateur hybride (1400) selon la revendication 4, dans lequel un premier élément de raidissement (1420) est un tube hypodermique et un second élément de raidissement (1420) est au moins un fil de raidissement.

6. Dilatateur hybride (1400) selon l'une quelconque des revendications 1 à 5, dans lequel le dilatateur hybride (1400) comprend en outre une couche de couple (1430).

7. Dilatateur hybride (1400) selon la revendication 6, dans lequel la couche de couple (1430) est une matière tressée,

8. Dilatateur hybride (1400) selon l'une quelconque des revendications 1 à 7, dans lequel la partie d'extrémité distale pouvant être orientée comprend au moins un anneau de traction (1482) qui est lié à l'au moins un fil de traction (1480).

9. Dilatateur hybride (1400) selon la revendication 8, dans lequel le dilatateur hybride (1400) comprend deux fils de traction (1480) et la partie d'extrémité distale pouvant être orientée comprend deux anneaux de traction (1482), soit un premier anneau de traction (1482) qui est lié à un premier fil de traction (1480) et un second anneau de traction (1482) qui est distal par rapport au premier anneau de traction (1482) et qui est lié à un second fil de traction (1480).

10. Dilatateur hybride (1400) selon la revendication 9, dans lequel la poignée comprend un premier moyen de commande de poignée (1472) qui est connecté de manière opérationnelle au premier fil de traction (1480) pour commander la direction de la partie d'extrémité distale pouvant être orientée et un second moyen de commande de poignée (1472) qui est connecté de manière opérationnelle au second fil de traction (1480) pour commander la direction de la partie d'extrémité distale pouvant être orientée (1460).

11. Dilatateur hybride (1400) selon l'une quelconque des revendications 1 à 10, dans lequel l'élément de raidissement (1420) se termine au niveau d'une extrémité distale de la partie proximale (1450).

12. Dilatateur hybride (1400) selon l'une quelconque des revendications 1 à 11, dans lequel l'arbre de dilatateur présente un diamètre externe compris entre environ 12 unités Charrière et environ 20 unités Charrière,

13. Dilatateur hybride (1400) selon l'une quelconque des revendications 1 à 12, dans lequel l'arbre de dilatateur comprend une couche externe (1414) et une couche interne (1412).

14. Dilatateur hybride selon la revendication 13, dans lequel la couche externe (1414) est fixée à la couche interne (1412) et dans lequel l'élément de raidissement (1420) est positionné entre la couche interne (1412) et la couche externe (1414).

15. Dilatateur hybride selon la revendication 14, dans lequel la couche externe (1414) est fixée à la couche interne (1412) au moyen d'un procédé de refusion.
